# EUROPEAN PATENT SPECIFICATION

(11) **EP 0 873 354 B1**
(45) Date of publication and mention of the grant of the patent: **07.12.2005**
(21) Application number: 97902865.1
(22) Date of filing: 07.01.1997
(51) Int. Cl.: C07K 2/00, C07K 14/47, C07K 16/18, C12N 5/10, C12N 15/09, C12N 15/11, C12N 15/12, C12N 15/63, C12N 15/70, C12N 15/74, C12N 15/79

(54) **NOVEL AMP ACTIVATED PROTEIN KINASE**
NEUARTIGE AMP-AKTIVIERTE PROTEINKINASE
NOUVELLES PROTEINES KINASES ACTIVEES PAR L'AMP

(30) Priority: 08.01.1996 AU PN745096
(43) Date of publication of application: 28.10.1998
(73) Proprietor: ST. VINCENT'S INSTITUTE OF MEDICAL RESEARCH, Fitzroy, VIC 3065 (AU); Trustees of Dartmouth College, Hanover, NH 03755-1404 (US)
(72) Inventor: KEMP, Bruce, E., Kew, VIC 3101 (AU); STAPLETON, David, I., Wantirna, VIC 3183 (AU); MITCHELHILL, Kenneth, I., East St. Kilda, VIC 3183 (AU); WITTERS, Lee, A., Norwich, VT 05055 (US)
(74) Representative: Stebbing, Peter John Hunter
(86) International application number: PCT/US1997/000270
(87) International publication number: WO 1997/025341

(56) References cited:
- WO-A-94/28116
- DATABASE EBI [Online] ID: HS79922; Accession number T50799, 26 February 1995 (1995-02-26) XP002155086
- DAVIES S.P. ET AL.: "Purification of the AMP-activated protein kinase on ATP-gamma-sepharose and analysis of its subunit structure" EUROPEAN JOURNAL OF BIOCHEMISTRY, vol. 223, 1994, pages 351-357, XP000972543
- CARLING D. ET AL.: "Purification and characterization of the AMP-activated protein kinase" EUROPEAN JOURNAL OF BIOCHEMISTRY, vol. 186, 1989, pages 129-136, XP000972542
- GAO G. ET AL.: "Non-catalytic beta- and gamma-subunit isoforms of the 5'-AMP-activated protein kinase" THE JOURNAL OF BIOLOGICAL CHEMISTRY, vol. 271, no. 15, 12 April 1996 (1996-04-12), pages 8675-8681, XP002181599
- STAPLETON D. ET AL.: "AMP-activated protein kinase isoenzyme family: subunit structure and chromosomal location" FEBS LETTERS, vol. 409, no. 3, 16 June 1997 (1997-06-16), pages 452-456, XP002181600
- J. BIOL. CHEM., 26 April 1996, Vol. 271, No. 5, WOODS A. et al., "Characterization of AMP-activated Protein Kinase beta and gamma Subunits", pages 10282-10290, XP002907303.
- J. BIOL. CHEM., 15 April 1994, Vol. 269, No. 15, CARLING D. et al., "Mammalian AMP-activated Protein Kinase is Homologous to Yeast and Plant Protein Kinases Involved in the Regulation of Carbon Metabolism", pages 11442-11448, XP002907304.
- J. BIOL. CHEM., 12 January 1996, Vol. 271, No. 2, STAPLETON D. et al., "Mammalian AMP-activated Protein Kinase Subfamily", pages 611-614, XP002907305.
- FEBS LETT., 1994, Vol. 356, BERI R.K. et al., "Molecular Cloning, Expression and Chromosomal Localisation of Human AMP-Activated Protein Kinase", pages 117-121, XP002907312.
- J. BIOL. CHEM., 25 November 1994, Vol. 269, No. 47, STAPLETON D. et al., "Mammalian 5'-AMP-activated Protein Kinase Non-catalytic Subunits are Homologs of Proteins that Interact with Yeast Snf1 Protein Kinase", pages 29343-29346, XP002907306.
- J. BIOL. CHEM., 28 January 1994, Vol. 269, No. 4, MITCHELHILL K.I. et al., "Mammalian AMP-activated protein Kinase Shares Structural and Functional Homology with the Catalytic Domain of Yeast Snf1 Protein Kinase", pages 2361-2364, XP002907307.
- EMBO J., 1994, Vol. 13, No. 24, YANG X. et al., "A Family of Proteins Containing a Conserved Domain that Mediates Interaction with the Yeast SNF1 Protein Kinase Complex", pages 5878-5886, XP002907322.
- BIOCHIM. BIOPHYS. ACTA, 1995, Vol. 1266, GAO G. et al., "Catalytic Subunits of the Porcine and Rat 5'-AMP-activated Protein Kinase are Members of the SNF1 Protein Kinase Family", pages 73-82, XP002907316.
- MOL. CELL. BIOL., November 1989, Vol. 9, No. 11, CELENZA J.L. et al., "Molecular Analysis of the SNF4 Gene of Saccharomyces Cerevisiae: Evidence for Physical Association of the SNF4 Protein with the SNF1 Protein Kinase", pages 5045-5054, XP000910655.
- MOL. BRAIN RES., 1996, Vol. 40, PIOSIK P.A. et al., "Carpine Homologue of Rodent 5'-AMP-activated Protein Kinase Subunit and Yeast SNF4/CAT3 is Down-regulated by Thyroid Horomone", pages 240-253, XP002907310.
- GENE, 1994, Vol. 149, AGUAN K. et al., "Characterization and Chromosomal Localization of the Human Homologue of a Rat AMP-activated Protein Kinase-Encoding Gene: a Major Regulator of Lipid Metabolism in Mammals", pages 345-350, XP002907323.
- FEBS LETT., 1995, Vol. 361, DALE S. et al., "Similar Substrate Recognition Motifs for Mammalian AMP-activated Protein Kinase, Higher Plant HMG-CoA Reductase Kinase-A, Yeast SNF1 and Mammalian Calmodulin-Dependent Protein Kinase I.", pages 191-195, XP002907311.
- J. BIOL. CHEM., 26 July 1996, Vol. 271, No. 30, DYCK J.R.B. et al., "Regulation of 5'-AMP-activate Protein Kinase Activity by the Noncatalytic beta and gamma Subunits", pages 17798-17803, XP002907308.
- EUR. J. BIOCHEM., 1995, Vol. 228, VERHOEVEN A.J.M. et al., "The AMP-activated Protein Kinase Gene is Highly Expressed in Rat Skeletal Muscle", pages 236-243, XP00240329
- J. BIOL. CHEM., 08 November 1996, Vol. 271, No. 45, MICHELL B.J. et al., "Isoform-specific Purification and Substrate Specificity of the 5'-AMP-activated Protein Kinase", pages 28445-28450, XP002907309.

## Description

### Background of the Invention

The 5'-AMP-Activated protein kinase (AMPK) was initially identified as a protein kinase regulating HMG-CoA reductase (Ferrer et al. (1985) *Biochem. Biophys. Res. Commun.* 132, 497-504). Subsequently, the AMPK was shown to phosphorylate hepatic acetyl-CoA carboxylase (Carling et al. (1987) *FEBS Lett.* 223, 217-222) and adipose hormone-sensitive lipase (Garton et al. (1989) *Eur. J. Biochem.* 179, 249-254). The AMPK is therefore thought to play a key regulatory role in the synthesis of fatty acids and cholesterol.

The AMPK is believed to act as a metabolic stress-sensing protein kinase switching off: biosynthetic pathways when cellular: ATP levels are deleted and when 5' -AMP rises in response to fuel limitation and/or hypoxia (Corton et al. (1994) *Current Biology* 4, 315-324). Partial amino acid sequencing of hepatic AMPK (Mitchelhill et al. (1994) *J. Biol. Chem.* 269, 2361-2364, Stapleton et al. (1994) *J. Biol. Chem.* 269, 29343-29346) revealed that it consists of 3 subunits, the catalytic subunit α (63 kDa), and two non-catalytic subunits, β (40 kDa) and γ (38 kDa).

The AMPK is a member of the yeast SNF1 protein kinase-subfamily that includes protein kinases present in plants, nematodes and humans. The AMPK catalytic subunit, α, has a strong sequence identity to the catalytic domain of the yeast protein kinase SNF1, which is involved in the induction of invertase (SUC2) under conditions of nutritional stress due to glucose starvation (Celenza, J.L. and Carlson, M. (1986) *Science* 233, 1175-1180). Both snf1p and the AMPK require phosphorylation by an activating protein kinase for full activity. The sequence relationship between snf1p and AMPK led to the finding that these enzymes share functional similarities, both phosphorylating and inactivating yeast acetyl-CoA carboxylase (Woods et al. (1994) *J. Biol. Chem*. 269, 19509-19516; Witters, L.A. and Watts, T.D. (1990) *Biochem. Biophys. Res. Commun.* 169, 369-376). The non-catalytic β and γ subunits of AMPK are also related to proteins that interact with snf1p; the β subunit is related to the SIP1/ SIP2 /GAL83 family of transcription regulators and the γ subunit to SNF4 (also called CAT-3) (Yang et al. (1994) *EMBO J.* 13, 5878-5886).

An isoform of the mammalian AMPK catalytic subunit has previously been cloned (Carling et al. (1994) *J. Biol. Chem.* 269, 11442-11448) and is referred to herein as AMPK α₂. The sequence of AMPK is disclosed in WO 94/28116. The AMPK α₂ does not complement SNF1 in yeast, indicating that their full range of functions are not identical.

A novel isoform of the mammalian AMPK catalytic subunit has now been identified and is referred to herein as AMPK α₁. In addition, full-length cDNAs for the mammalian AMPK β and AMPK γ subunits have now been cloned and polypeptides encoded thereby purified.

### Summary of the invention

According to a first aspect of the present invention there is provided a nucleic acid sequence encoding mammalian AMPK α₁, the nucleic acid sequence comprising SEQ ID NO:44.

Further provided is a vector comprising the nucleic acid sequence.

A host cell comprising the vector is also provided.

In further embodiments a recombinant polypeptide encoded by the nucleic acid sequence is provided.

According to a further aspect of the present invention there is provided a method of producing mammalian AMPK α₁ comprising:
(a) culturing the host cells under conditions which allow expression of the nucleic acid sequence encoding mammalian AMPK α₁; and
(b) recovering the expressed AMPK α₁ from the cell.

According to another aspect of the present invention, there is provided an oligonucleotide probe comprising at least 10 nucleotides, said oligonucleotide probe being capable of hybridizing to the nucleic acid sequence, having SEQ ID NO: 67, which encodes amino acids 352-366 of AMPK α₁, but not to a nucleic acid sequence encoding a mammalian AMPK α₂ polypeptide encoded by nucleic acid molecules having sequences of SEQ ID NO: 65 or 66.

Further provided is a purified polypeptide or biologically active fragment thereof encoded by the nucleic acid sequence, wherein, said biologically active fragment is not present in a mammalian AMPK α₂ polypeptide encoded by nucleic acid molecules having sequences of SEQ ID NO: 65 or 66 and comprises:
(a) at least a portion of SEQ ID NOs: 1-12, 15-32, 35-38, 40 or 43
(b) SEQ ID NOs: 14, 34, 39, 41 or 42
(c) retains the ability to stimulate phosphorylation of protein molecules; or
(d) retains the ability to mimic the binding of native AMPK α₁ polypeptide to at least one antibody or substrate molecule.

Accordingly, the present invention relates to an isolated polynucleotide which encodes mammalian AMPK α₁ or a sequence which hybridizes thereto with the proviso that the sequence does not hybridize to mammaliam AMPK α₂ as defined in Table 1 (SED ID NO: 65)or Table 5 (SEQ ID NO: 66) of WO 94/28116.

It will be understood by those of skill in the art that the oligonucleotide probes according to the present invention may be used in a number of procedures. These include the analysis of gene regulatory elements; the analysis of gene expression *in vivo*; and the identification of homologous mammalian and non-mammalian cDNAs including the associated kinase-kinase.

By "biologically active fragment" it is meant a fragment which retains at least one of the activities of native AMPK α₁ which activities include (i) the ability to stimulate phosphorylation of protein molecules; and (ii) the ability to mimic the binding of native AMPK α₁ to at least one antibody or substrate molecule.

It will be appreciated by those skilled in the art that a number of modifications may be made to the polypeptides and fragments of the present invention without deleteriously effecting the biological activity of the polypeptides or fragments. This may be achieved by various changes, such as sulfation, phosphorylation, nitration and halogenation; or by amino acid insertions,: deletions and substitutions, either conservative or non-conservative (e.g., D-amino acids, desamino acids) in the peptide sequence where such changes do not substantially alter the overall biological activity of the peptide. By conservative substitutions the intended combinations are: G,A; V,I,L,M; D,E;:N,Q; S,T; K,R,H; F,Y,W,H; and P, Nα -alkylamino acids.

It is also possible to add various groups to the polypeptides or fragments of the present invention to confer advantages such as increased potency of extended half-life in vivo, without substantially altering the overall biological activity of the peptide.

The mammalian AMPK α₁ polypeptide of the present invention may be used to identify compounds which regulate the action of this kinase. Such compounds are desirable since, for example, they may be used to reduce the biosynthesis of cholesterol and fatty acids. They may also be used to inhibit the release of these from intracellular stores: by HSL. In addition, they may be used the reduce cellular malonyl CoA levels and promote the β-oxidation of fatty acids by the mitochondria.

Compounds may be screened for mammalian AMPK α₁ antagonist: or agonist activity by exposing mammalian AMPK α₁ of the present invention to the compounds and assessing the activity of the mammalian AMPK α₁. Suitable screening methods for identifying compounds which regulate the activity of mammalian AMPK α₁ include any conventional assay systems for determining such effects.

In addition, compounds may be screened: for ability to regulate expression of mammalian AMPK α₁ in a cell by exposing the cell transformed with the polynucleotide: of SEQ ID:NO: 44 to: the compound and assessing the level of expression of the polynucleotide.

Suitable screening methods for identifying compounds which regulate expression of mammalian AMPK α₃ include those which involve the detection and/or determination of the amount of mammalian AMPK α₁ or messenger RNA that encodes mammalian AMPK α₁ or protein in the presence of the relevant test compound.

As indicated above, the compounds which regulate activity of mammalian AMPK α₁ are considered to be of potential use in the treatment of, for example, hypercholesterolemia, hyperlipidemia, obesity, clinical syndromes associated with hypoxia or ischemia (e.g., myocardial infarction, stroke), disorders of nutrition and diabetes mellitus.

Full-length cDNAs for the mammalian AMPK β and AMPK γ subunits have now been cloned. These clones have been used to characterize the tissue distribution of subunit mRNA and to express subunit protein in both bacteria and mammalian cells. Identification of their complete sequences has also led to the identification of protein families: for each of these non-catalytic subunits.

The present invention will now be described with reference to the following detailed description. In this connection, the present invention only relates to the subject matter included in the claims.

Mammalian AMPK, as isolated from rat and porcine liver, contains three polypeptide subunits, termed AMPK α, AMPK β and AMPK γ. The α subunit contains the kinase catalytic domain sequence and is highly homologous to several members of the SNF1 kinase family. Multiple isoforms of the α subunit have now been identified with α₁ being responsible for about 90% of the AMPK activity detected in liver extracts. In addition,: it has now been established that full-length AMPK β and AMPK γ subunits are likewise homologous to two classes of proteins in *S. cerevisiae.* This extends information previously available from limited peptide sequence analysis and from smaller PCR-derived cDNAs (Stapleton et al. (1994) *J. Biol*. *Chem.* 269, 29343-29346). Further, by cDNA cloning and direct peptide sequencing is has been demonstrated which isoforms of AMPK β and AMPK γ subunits interact with the α₁ catalytic subunit in liver. Thus, is has now been found that these non-catalytic subunits, like the α subunit isoforms, have a wider tissue distribution, as evidenced by mRNA content of several rat tissues, than expected from the AMPK activity distribution previously reported by Gao et al. (1995) *Biochem. Biophys. Acta.* 1200, 73-82 and Davies et al. (1989) *Eur*. *J. Biochem*. 186, 123-128.

An isoform of the mammalian AMPK catalytic subunit has been identified and is referred to herein as AMPK α₁. The α₁ (548 residues) and α₂ (552 residues) isoforms of AMPK have 90% amino acid sequence identity within the catalytic core but only 61% elsewhere. The major differences in the α₁ and α₂ sequences occur in their COOH-terminal tails which suggests that they may interact with different proteins within this region.

It has now been found that the *α*₂ 8.5 kb mRNA is most abundant: in skeletal muscle with lower levels in liver, heart and kidney. In contrast, very low levels of the α₁ 6 kb MRNA were found in all tissue except testis, where a low level of an uncharacterized 2.4 kb mRNA was observed. The low levels of α₁ mRNA explains why the α₁ isoform was more difficult to clone than the α₂ isoform. The α₁ isoform of the AMPK catalytic subunit, however, accounts for approximately 94% or more of the SAMS peptide phosphotransferase activity of rat liver and is therefore the predominant active expressed hepatic isoform.

A series of synthetic peptides including analogues of proteins not known to be substrates for the AMPK were screened with partially purified enzyme (purified to the DE-52 step). These included the myosin light chains, ADR1, glycogen synthase and phospholemman. The phospholemman peptides tested were poor substrates and not investigated further. The glycogen synthase peptide, PLSRTLSVAAKK (SEQ ID NO: 46) was phosphorylated in an AMP-dependent manner at approximately 40% of the rate of the SAMS peptide, however, this peptide is an excellent substrate for a number of protein kinases, including protein kinase C and calmodulin dependent protein kinase II (Kemp, B.E. and Pearson, R.B. (1991) in Protein Phosphorylation, Hunter, T and Sefton, B.M. (eds) Methods in Enzymology, 200, 121-134). The myosin light chain peptides tested were phosphorylated with rates approximately 15% of the SAMS peptide. It was found that the ADR1 peptides ADR1 (225-234) and ADR1 (222-234)^{P229} were phosphorylated at rates of approximately 50% of the SAMS peptide. Results from these experiments indicate that the ADR1 (222-234)^{P229} peptide is phosphorylated with an apparent Km of approximately 3 µM compared to 33 µM for the SAMS peptide.

In view of the low Km of the ADR1 (222-234)^{P229} peptide as a substrate for the AMPK, affinity purification of the enzyme with this peptide was attempted. Initially the peptide was coupled to CNBr-activated sepharose. Although the peptide linked sepharose bound the AMPK containing fractions the enzyme could not be differentially eluted from contaminating proteins with salt gradients. In contrast when the ADR1 (222-234)^{P229} peptide was coupled to Pharmacia HiTrap column the AMPK was bound very tightly and required 2 M NaCl plus 30% ethylene glycol to elute it. Because the enzyme bound so tightly to this substrate affinity column it was possible to load the enzyme in buffer containing 0.5 M NaCl. The resultant purified AMPK consisted of a 63 kDa catalytic subunit and 40 kDa and 38 kDa subunits related to sip2 and snf4, respectively. In some preparations the AMPK was associated with high molecular weight material that corresponded to non-muscle myosin as assessed by tryptic peptide sequencing. An apparent purification of up to 38,000 with a yield of 15% and a recovery of 90 µg of enzyme was obtained. The fold purification may be an overestimate due to the presence of uncharacterized inhibitory material in the early fractions. The enzyme was not apparent on SDS-PAGE until the final step of purification. The avidity of the enzyme for the peptide bound to the Pharmacia HiTrap resin was greater than could be expected from the free peptide binding to the enzyme (Km 3 µM). Since the peptide linked to sepharose did not bind the enzyme as tightly it seems reasonable that the enhanced binding is due in part to the aminohexanoic acid linker between the peptide and the resin. In the case of the cAMP-dependent protein kinase there is a hydrophobic pocket between the D and G helices that is responsible for high affinity binding of the peptide inhibitor PKI. Since the ADR1(222-234)*P229* peptide, LKKLTLRASFSAQ (SEQ ID NO: 47), is linked through the amine residues on its N-terminus or Lys residues, it is possible that the hydrophobic linker group has been fortuitously juxtaposed to a hydrophobic pocket on the AMPK.

In the course of sequencing the porcine AMPK it was found that the amino acid sequence of some peptides derived from the pig liver AMPK α subunit did not match those deduced from the rat liver cDNA sequence (Carling et al. (1994) *J. Biol. Chem.* 269, 11442-11448; Gao et al. (1995) *Biochem. Biophys. Acta.* 1200, 73-82). Therefore, the rat liver AMPK catalytic subunit, α was purified and peptides accounting for 40% of the protein sequenced (222/548 residues, SEQ ID NOs: 27-43). Eight of the 16 peptides contained mismatched residues with the reported AMPK cDNA sequence, but did match the pig liver enzyme sequence (SEQ ID NOs: 13-26). Using RT-PCR and cDNA library screening, a cDNA sequence of the rat hypothalamus enzyme was obtained that accounted for all of the peptide sequences of the purified rat liver AMPK catalytic subunit containing mismatches. The cDNA sequence of this AMPK catalytic subunit has been named α₁, since it corresponds to the purified enzyme and is clearly derived from a different gene than the previously cloned a sequence (now referred to as α₂). The α₁ isoform of the AMPK catalytic subunit accounts for approximately 94% or more of the SAMS peptide phosphotransferase activity of rat liver and is therefore the predominant active expressed hepatic isoform. Despite sequencing multiple preparations of the AMPK catalytic subunit from both pig and rat liver (SEQ ID NOs 13-26 and 27-43, respectively), no peptides were obtained that matched the α₂ isoform sequence.

Within the catalytic cores of the α₁ and α₂ isoforms, there is 90% amino acid identity but only 61% identity outside the catalytic core. Strong homology between the α₁ and α₂ sequences in the vicinity of the substrate binding groove, inferred from the protein kinase crystal structure for positions P₋₅ to P₊₅, suggest that the substrate specificities will be related. The substrate anchoring loop (also called the lip or activation loop) contains an insert FL¹⁷⁰ for α₁, α₂ and snf1p that may provide a hydrophobic anchor for a P₊₃ or P₊₄ hydrophobic residue in the peptide substrate. There is also E¹⁰⁰ (E¹²⁷ in cAMP-dependent protein kinase) and D¹⁰³ available for a P₋₃ basic residue specificity determinant for both the α₁, α₂ and snf1p. Both isoforms contain a Thr-172 residue equivalent to Thr-197 in the cAMP-dependent protein kinase, which is likely to be phosphorylated and necessary for optimal activity. Since the major differences in the α₁ and α₂ sequences occur in their COOH-terminal tails they may interact with different proteins within this region.

Northern blot analysis of the β and γ subunits revealed a complex pattern of expression. The β subunit mRNA was least abundant with similar levels across a range of tissues except brain, whereas the γ subunit mRNA was abundant in heart, lung, skeletal muscle, liver and kidney. An earlier report on the tissue distribution of the AMPK activity had claimed that it was predominantly a liver enzyme (Davies et al. (1989) *Eur. J. Biochem.* 186, 123-128). In view of the mRNA distribution of the α₁ and β subunits, the tissue distribution of the AMPK activity was reassessed. The kidney contained the highest specific activity with similar levels in the liver, lung and heart and little, if any, activity in skeletal muscle. It is clear that the AMPK activity has a wider tissue distribution than appreciated heretofore, closely paralleling the distribution of α₁ mRNA and not that of α₂ mRNA. Using peptide specific antisera to α₁ (residues 339-358) and α₂ (residues 352-366) it was found that the α₂ immunoreactivity was predominant in the heart, liver and skeletal muscle where there is also the highest concentrations of *α*₂ mRNA. In contrast the α₁ immunoreactivity is widely distributed as is the less abundant α₁ mRNA. The antibody to α₂ recognized a minor component in the purified α₁ preparation but sufficient amounts of this have not been obtained to determine whether it represents weak cross reactivity with a form of α₁, an additional isoform of the AMPK or a low level contaminant of the α₁ preparation by the α₂ isoform. The antibody to α₂ does not immunoprecipitate α₁ activity from affinity purified α₁ AMPK. Both α₁ and α₂ migrate on SDS-PAGE at approximately 63 kDa. It was also found that the liver α₂ immunoreactivity was not bound by the peptide substrate affinity column. This column specifically binds the α₁ isoform. Using immune precipitation of the effluent from the peptide substrate affinity column with α₂ specific antibody it was found that the α₂ isoenzyme contained β and γ subunits and catalyzed the phosphorylation of the SAMS peptide. Immune precipitates of α₁ and α₂ showed variable activation by 5'-AMP ranging from 2-3 and 3-4 fold, respectively. There was also an approximate 60 kDa band recognized by the α₁-specific antibody in tissue extracts from heart and lung. This band is not present in the purified liver enzyme and its relationship to the α₁ isoform is not yet known.

The proportion of SAMS peptide phosphotransferase activity bound to the peptide affinity column with a single pass varied (ranged 90-92%, n=7 and 74-86%, n=6 rat liver preparations). With recycling, approximately 94% of the activity was bound to the column. The residual activity was attributable to α₂ isoform activity based on immunoprecipitation with the α₂-specific antibody. However, the amount of protein immunoprecipitated based on Coomassie blue staining indicated that there was substantially more α₂ protein than was expected from only 6% of the total SAMS peptide activity. The apparent specific activity of the isolated rat hepatic AMPK α₂ isoform with either the SAMS peptide or acetyl CoA carboxylase as substrate was more than 20-fold lower than the AMPK α₁ isoform. This estimate is based on measurements using the α₂ enriched fraction (α₁ depleted) and quantitation by immunoblotting compared to bacterially expressed α₂.

The specific activity of the purified α₂ isoform is not yet known in the absence of bound antibody. Based on the α₂ cDNA sequence, Carling et al. (1994) *J. Biol. Chem.* 269, 11442-11448 reported that a peptide specific antibody immunoprecipitated virtually all of the partially purified AMPK activity from liver. The peptide used in their experiments, PGLKPHPERMPPLI (SEQ ID NO: 48), contains 8/15 residues that are identical (underlined) between α₁ and α₂ so it seems reasonable that their polyclonal antisera may recognize both isoforms.

These experiments make clear that there is an isoenzyme family of AMPK α catalytic subunits, thus increasing the complexity of activity analysis. This also raises the question of what function the α₂ isoform has and whether α₂ associates with a specific subset of β and γ subunits. A significant fraction of the α₂ isoform mRNA has a 142 bp out-of-frame deletion within its catalytic domain that would encode a truncated, non-functional protein (Gao et al. (1995) *Biochem. Biophys. Acta.* 1200, 73-82; Verhoeven et al. (1995) *Eur. J. Biochem.* 228, 236-243). The close sequence relationship between the α₁ isoforms from pig, rat and human means that there is strong conservation across species. Previously, it was reported that human liver does not contain AMPK mRNA (Aguan et al.(1994) *Gene* 149, 345-350). However, it is now clear that α₂ mRNA was being probed for and not the dominant α₁ isoform mRNA. The gene encoding the human liver AMPK catalytic subunit reported on chromosome 1 is therefore the gene for the α₂ isoform whereas the gene for the α₁ isoform is located on chromosome 5. The AMPK subunit genes have now been mapped predominantly to the following chromosomal locations: α₁, 5p12; β, 5q24.1; and γ, 12q13.1.

Recent genome sequencing has revealed multiple isoforms of the non-catalytic γ and β subunits of the AMPK. There appear to be at least three isoforms of the γ subunit in brain with γ₂ and γ₃ present, distinct from the rat liver γ₁ isoform. Human brain also contains multiple β subunit isoforms distinct from the rat liver β₁ isoform. The accession numbers for putative AMPK β and γ subunit isoforms are γ₂, M78939; γ₃, R52308; β₂, R20494 and β₃, R14746. Thus, a potentially large subfamily of heterotrimeric AMPKs, based on various combinations of all three AMPK subunits, may be present.

The structural relationships between the AMPK and SNF1 kinase, as well as the presence of multiple isoforms, brings into focus a vista of questions concerning the diverse physiological roles of this new subfamily of protein kinases. Whereas the AMPK regulates lipid metabolism in hepatocytes under conditions of metabolic stress, its role in other tissues, including the heart and kidney, are unknown. Recent studies have shown that the AMPK is activated during cardiac ischaemia, and the activation persists during reperfusion, possibly contributing to the ischaemia-driven decoupling of metabolism and cardiac mechanical function (Kudo et al. (1995) *J. Biol. Chem.* 270, 17513-17520).

Regulation of cardiac acetyl-CoA carboxylase by AMPK plays an important role in the switching of cardiac metabolism between the use of glucose and fatty acids as oxidative fuel. In the β cell of the pancreas, where AMPK subunits are highly expressed in islet cells, glucose availability rapidly regulates acetyl-CoA carboxylase through changes in AMPK-directed phosphorylation, suggesting strongly a role for AMPK in stimulus-secretion coupling for insulin release. In addition to these metabolic roles, members of the SNF1 protein kinase subfamily appear to play important roles in development, with the par-1 gene of *C. elegans* playing an essential role in embryogenesis.

PCR amplification of pig and rat liver cDNA with degenerate oligonucleotides corresponding to selected AMPK β peptide sequences yielded two major PCR products (Stapleton et al. (1994) *J. Biol. Chem.* 269, 29343-29346). One product, a rat 309 bp partial length cDNA, was used to screen a rat liver cDNA library, yielding a 1107 bp clone (SEQ ID NO: 61). The screening PCR probe corresponded to nt residues 279-588 of this sequence. This clone contains an open reading frame encoding for a 270 amino acid peptide (SEQ ID NO: 62), which contains all of the 15 independent (some overlapping) peptide sequences obtained from extensive sequence analysis of the purified protein. The translational start methionine codon is assigned from the typical Kozak sequence present for a initiation codon and the lack of any other upstream in-frame methionine codons. While no in-frame stop codon is present in this 5'-upstream sequence, a human expressed sequence tag (EST) cDNA (GenBank accession no. T78033) in the database contains such a stop codon preceding the same assigned methionine start. This reading frame, however, predicts a protein of 30,464 daltons, well below the estimated molecular weight of 40 kDa evident on SDS gel electrophoresis.

In order to clarify the size of the protein product that could be synthesized from this cDNA, the AMPK β clone was expressed both in bacteria and mammalian cells. In both expression systems, the protein product migrates at a higher than predicted molecular weight. When purified as a His⁶-tagged fusion protein from *E. coli*, the isolated protein migrates on SDS gels with an apparent molecular weight of about 43,000 Da (the same as the ovalbumin standard). This corresponds to a AMPK β polypeptide product of 40 kDa with an additional 3 kDa daltons of fusion tag sequence derived from the pET vector. When expressed in mammalian cells from an HA-tagged expression vector, two polypeptides are evident with the major product corresponding to a 40 kDa species (after correction for the size of the HA epitope tag). A second product of 42-43 kDa is also evident using this expression system. Taken together, these data demonstrate that the protein product of this AMPK β migrates on SDS-PAGE with an anomalously high molecular weight.

Comparison of the rat liver AMPK β sequence to the database reveals that it is highly homologous to three yeast proteins (Siplp, Sip2p and Gal83p) and to two recently cloned human EST-cDNA sequences. This alignment, as gapped according to the sequence of the *S. cerevisiase* protein, Sip1p (Yang et al. (1992) Science, 257, 680-682), is most striking at the C-terminus of AMPK β and these yeast proteins.

The AMPK β subunit is a mammalian homolog of a class of proteins in yeast, represented by Siplp/Sip2p/Gal83p. The GAL83 gene product is known to affect glucose repression of the GAL genes. All of these proteins have been shown to interact with the Snf1p protein kinase either in the 2-hybrid system or by immunoprecipitation. It has been proposed that these proteins serve as adaptors that promote the activity of Snf1p toward specific targets. Based on analysis of yeast mutants, it has been suggested that these proteins may facilitate interaction of Snf1p with unique and different targets. Of interest is the demonstration of a highly conserved domain of about 80 amino acids in the C-terminus of Sip1p/Sip2p/Ga183p, termed the ASC domain (association with Snf1p complex)(Yang et al. (1994) *EMBO J.* 13, 5878-5886). As studied in the 2-hybrid system, the ASC domain of both Sip1p and Sip2p interacts strongly with Snf1p. However, the interaction of Sip2p with Snf1p is not entirely lost on deletion of this domain, suggesting that the ASC domain is not solely responsible for this protein-protein interaction. A putative ASC domain is also highly conserved in the C-terminus of rat liver AMPK β (aa residues 203-270), suggesting that this region may be responsible, in part, for binding to the AMPK α subunit.

AMPK β, like Sip2p and Ga183p, is phosphorylated in vitro when associated with a catalytic subunit (AMPK a or Snf1p, respectively). Mutations of Gal83p can abolish most of the Snf1p kinase activity detectable in immune complexes, precipitated with anti-Snf1p antibody. A *Sip2p*/*E gal 83*/*E* mutant shows reduced Snfl protein kinase activity, that is restored following expression of either Sip2p or Gal83p LexA-fusion proteins in the mutant strain (Yang et al. (1994) *EMBO J.* 13, 5878-5886). Taken together, these data suggest the possibility that AMPK β may also serve as an adaptor molecule for the AMPK α catalytic unit and will positively regulate AMPK activity.

AMPK β appears to have anomalous migration on SDS gels, with the polypeptide migrating at a Mᵣ approximately 10 kDa larger than the size predicted from the cDNA. This slower migration is evident for both the bacterially expressed His⁶-fusion protein and for the protein expressed in COS7 cells. These observations suggest that higher orders of structure are responsible for the anomalous migration on SDS-PAGE. The AMPK β subunit is autophosphorylated *in vitro*; this suggests that the two AMPK β bands expressed on transfection of mammalian cells with AMPK β cDNA may result from a similar post-translational modification giving rise to smaller mobility shifts. Interestingly, this aberrant migratory behavior of AMPK β is similar to that of its yeast homolog, Gal83p. The LexA-fusion protein(s) of Ga183, as expressed in yeast, also migrate at greater than the expected molecular weight and display more than one band on SDS gels, consistent with the known phosphorylation of Ga183p by Snf1p. Mass spectrometry analysis of the β-subunit indicates that the amino terminal glycine is myristylated and that the subunit is isolated in mono- and di- phosphorylated forms.

Using the MOPAC procedure and other PCR amplification protocols, a 192 bp cDNA corresponding to rat liver AMPK γ sequence was obtained and used for library screening to obtain a partial length rat liver cDNA of approximately 1.3 kb. This sequence did not contain either a start methionine codon or all the peptide sequences obtained from the purified protein. Attempts to extend this sequence to the 5'-end by the use of a primer extension library and 5'-RACE only succeeded in adding about 200 nt to this sequence without identification of the start codon. A partial length rat cDNA was then used to screen a human fetal liver library, which did yield the full-length clone depicted in SEQ ID NO: 63. This clone contains a deduced amino acid sequence (SEQ ID NO: 64) corresponding to all of 22 independent (some overlapping) peptide sequences obtained from the purified rat and porcine liver AMPK γ, confirming clonal identity.

A typical Kozak translation initiation sequence surrounds the assigned methionine start codon; this start is also in-frame with a 5'-upstream stop codon. The assigned start methionine is followed by an open reading frame predicting a protein of 331 amino acids and of 37,546 Da, which corresponds to the molecular weight observed on SDS gel electrophoresis of the protein as purified from rat and porcine liver. Expression of a truncated rat AMPK γ cDNA (aa residues 33-331) and the full-length human AMPK γ (331 aa) in COS7 cells yields products consistent with the molecular weight predicted for each cDNA (34,081 and 37,577 daltons, respectively). The rat liver AMPK γ product expressed in bacteria also displayed the molecular weight predicted by the cDNA. Thus, unlike AMPK β, there is no anomalous migration of the protein product of AMPK γ cDNA.

Comparison of the human and rat liver AMPK γ amino acid sequences to the database yields a significant alignment of this protein with the *S. cerevisiae* Snf4p. In addition, human full-length cDNA of the present invention also aligns with several other human partial length EST-cDNA sequences from brain, breast, placenta, liver and heart, recently reported in the database. Inspection of these sequences reveals that there are multiple isoforms of the human AMPK γ protein. There are likely also similar AMPK γ isoform families expressed in the rat and pig. This latter expectation is based on sequence analysis of 14 other MOPAC-derived partial AMPK γ cDNA sequences, as identified on colony hybridization of the AMPK γ MOPAC products with ³²P-labeled degenerate oligonucleotides. These products showed at least two reproducible patterns of nucleotide heterogeneity within the non-degenerate core.

Rat and human liver AMPK γ is a mammalian homolog of the *S. cerevisiase* Snf4p (CAT3) (Celenza et al. (1989) *Mol. Cell. Biol.,* 9, 5045-5054; Schuller, H.J. and Entian, K.D. (1988) Gene, 67, 247-257; Fields, S. and Song, O.K. (1989) Nature, 340, 245-246). Snf4p was shown to interact with the Snf1p protein in the first reported use of the 2-hybrid system and also co-immunoprecipitates with it (Haygood, M.G. (1993) *Biotechniques* 15, 1084-1089). Indeed, on isolation of the Snf1p kinase from yeast, Snf4p, but not the other Snf1p-interacting proteins, co-purifies in a 1:1 stoichiometry with the Snf1p polypeptide. Analysis of SNF4 mutants in yeast suggests that Snf4p also positively regulates the activity of its associated catalytic subunit, Snf1p. By analogy, our prediction is that AMPK γ will also have such a positive influence on the AMPK α subunit.

Examination of the database reveals that, in addition to the homology of AMPK γ to Snf4p, there are 2 or 3 different human proteins highly homologous or identical to our human and rat liver AMPK γ sequences. However, some of these database sequences, as predicted from EST-cDNAs in brain, heart, breast and placenta, are distinct from each other and from our clones; some, for example, have a C-terminal extension. This indicates that there is a mammalian isoform family of potential AMPK γ subunits, each perhaps with different tissue expression and regulatory roles. It is suggests that these different gamma isoforms be designated γ₁, γ₂, γ₃ .... γₙ, as their full-length sequences are delineated. The rat liver/human liver AMPK γ sequence of the present invention is designated herein as AMPK γ₁.

AMPK α catalytic unit is widely expressed in several rat tissues. AMPK β and AMPK γ sequences have a similar wide tissue expression. Two species of AMPK γ mRNA of 2.7 and 1.9 kb are evident in total mRNA preparations; only the latter is present in polyA+-RNA from rat liver, suggesting that the larger mRNA is an unprocessed precursor. Only a single mRNA species for AMPK β of 1.9 kb is evident. Both AMPK γ and AMPK β mRNAs are highly expressed in kidney, white adipose tissue, lung and spleen, while AMPK γ mRNA appears to be more highly expressed in heart and brain. While detectable, the mRNA level for each subunit is relatively lower in skeletal muscle, lactating mammary gland and liver. In other studies, high concentrations of mRNA have been found for both subunits in the rat Fao hepatoma cell and the Syrian hamster insulin-secreting HIT cell, cell lines that both express substantial levels of AMPK activity.

AMPK was first recognized as a protein kinase active on enzymes of lipid metabolism (acetyl-CoA carboxylase, HMG Co-A reductase and hormone-sensitive lipase). However, as has been observed for the AMPK α subunit, the AMPK β and AMPK γ₁ subunits have wider tissue distribution than might be expected for a protein active only in the regulation of lipid metabolism. While mRNAs for each are detectable in "classic" lipogenic tissues like liver, white adipose tissue and lactating mammary gland, high concentrations of mRNA in non-lipogenic tissues like heart, brain, spleen and lung, for example, suggest that these proteins have roles that extend beyond the regulation of biosynthesis of fatty acids and sterols and fatty acid oxidation.

For example, the striking homology of all three subunits to yeast proteins that are involved in nutrient (glucose) responses suggests that the three mammalian proteins may be involved in glucose (or other nutrient) regulation of gene expression in mammalian tissues or in other adaptive responses to a changing nutrient environment. In addition, AMPK may be a important "metabolic sensor" linked to oxidative fuel choice in the heart and to glucose sensing in the pancreatic beta cell, perhaps being important for insulin secretion.

The following nonlimiting examples are provided to further illustrate the instant invention.

### EXAMPLES

### Example 1: Purification of AMPK Catalytic Subunit (α1)

### Enzyme Purification

AMPK was purified from porcine liver. Liver (1 kg) was homogenized in 4,000 ml of buffer. A 2.5-7.0% (w/v) PEG 6000 fraction was prepared and the resultant fraction batched onto 1,500 ml of DEAE cellulose (Whatman, Clifton, NJ) and eluted with buffer containing 0.25 M NaCl (2,000 ml). The eluate was chromatographed on 150 ml Blue Sepharose (Pharmacia, Uppsala, Sweden) and the AMPK eluted with buffer containing 1 M NaCl. The enzyme fraction was concentrated and desalted by 10% (w/v) PEG-6000 precipitation prior to chromatography by peptide substrate affinity chromatography. The peptide substrate affinity column was washed with the same buffer containing 0.1% (v/v) Triton X-100 and 0.5 M NaCl and the AMPK eluted with this buffer containing 2 M NaCl and 30% (v/v) ethylene glycol.

### Protein kinase assays

The AMPK was assayed in accordance with procedures described by Davies et al. (1989) *Eur. J. Biochem.* 186, 123-128 using the SAMS peptide substrate, HMRSAMSGLHLVKRR-amide (SEQ ID NO: 49). The enzyme was diluted in diluting buffer (20 mM HEPES pH 7.0, 0.1% (v/v) Triton X-100) prior to assay and the reactions were initiated by adding 10 ml diluted enzyme to the reaction mixture containing peptide substrate. The reactions were stopped by withdrawing 30 ml aliquots and applying to P81 papers in accordance with procedures described by Pearson, R.B., Mitchelhill, K.I., and Kemp, B.E. (1993) in Protein *Phosphorylation: A Practical Approach,* Hardie, G.D. (ed) Oxford University Press, pp 265-291.

### Peptide synthesis

Peptides were synthesized using an Applied Biosystems 430 synthesizer in accordance with procedures described by Pearson, R.B., Mittchelhill, K.I., and Kemp, B.E. (1993) in *Protein Phosphorylation: A Practical Approach,* Hardie, G.D. (ed) Oxford University Press, pp 265-291. All peptides were purified by cation-exchange chromatography followed by reverse phase chromatography. Peptides were analyzed by quantitative amino acid analysis using a Beckman 6300 amino acid analyzer. The peptide substrate affinity column was prepared by coupling the ADR1 (222-234)^{P229}, peptide to a Pharmacia HiTrap N-hydroxysuccinamide ester activated superose column. This resin contains a 6-aminohexanoic acid spacer arm. The conditions of coupling were performed in accordance with manufacturer's instructions with 10 mg peptide per 5 ml column and peptide coupling was monitored by reverse phase HPLC.

### Example 2: Isolation of cDNA Encoding AMPK Catalytic Subunit (α1)

### Peptide Sequencing

Peptides were derived from rat and porcine α1 subunit of the AMPK, by *in situ* proteolysis in accordance with procedures described by Mitchelhill et al. (1994) *J. Biol. Chem.* 269, 2361-2364 and sequenced on either an Applied Biosystems 471A Protein Sequencer or a Hewlett Packard G1000A Protein Sequencer.

### Tissue Distribution Activity Studies

A 35% saturated ammonium sulfate fraction was prepared for each tissue, following homogenization in AMPK homogenization buffer (HB, 50 mM Tris-HCl pH 8.5, 250 mM sucrose, 5 mM sodium pyrophosphate, 50 mM sodium fluoride, 1 mM EGTA, 1 mM EDTA, 1 mM DTT, 1 mM benzamidine, 1 µg/ml soybean trypsin inhibitor and 0.2 mM phenylmethyl-sulfonylfluoride). The resultant pellet was resuspended in 5 ml HB and assayed for protein concentration. The AMPK was assayed in accordance with procedures described by Mitchelhill et al. (1994) *J*. *Biol*. *Chem.* 269, 2361-2364 with the following modifications: a final reaction volume of 120 µl was used, enzyme aliquots (30 µl) containing 1 µg protein pre-diluted in 50 mM Tris-HCl pH 7.5 and 0.05% (v/v) Triton X-100 were used to initiate the reaction. Three aliquots (30 µl) were removed after 2, 4 and 6 min. Reactions were performed in duplicate ± 5'-AMP (200 µM), with a minus peptide substrate control. The specific activity of the enzyme was determined using linear rates of phosphorylation with the specific synthetic peptide substrate SAMS. The AMPK was purified from rat or porcine liver as described in Example 1 using substrate affinity chromatography.

### Isolation of AMPK cDNA

A radiolabelled cDNA (774bp) encoding porcine AMPK α₁ was used to screen a rat hypothalamus Zap II cDNA library (Stratagene, La Jolla, CA) according to the manufacturer's instructions. Positives were plaque-purified on subsequent rounds of screening and phagemid from positive clones were rescued with helper phage (Stratagene). Screening of 7x10⁶ plaques yielded three unique clones, the largest consisting of an open reading frame, corresponding to AMPK α₁ (2-549).

The AMPK α₁ 5' end was isolated using a Gibco 5'-RACE kit (Life Technologies, Grand Island, USA) with an α₁ specific primer to residues 41-48 and rat liver cDNA. Human AMPK α₁ (14-270) was isolated from fetal human liver cDNA primed with sense and anti-sense partially degenerate oligonucleotides to α₁ peptide sequence by RT-PCR. Human AMPK α₁, residues 291-448 is a partial length human liver cDNA clone obtained from the Lawrence Livermore National Laboratory (clone 78297, accession number T50799).

### Northern Blotting

A rat multiple tissue Northern (MTN) blot (Clontech, Palo Alto, CA, USA) containing 2 mg of poly (A) + RNA of individual tissues was probed with ³²P-labelled rat AMPK α₁ and α₂ cDNAs according to the instructions supplied.

### Production of Anti-AMPK Antibodies

Polyclonal antibodies to AMPK α₁ and α₂ were prepared as follows. Peptides based on the predicted amino acid sequences of AMPK α₁ for residues 339-358 (DFYLATSPPDSFLDDHHLTR (SEQ ID NO: 50)) and AMPK α₂ for residues 352-366 (MDDSAMHIPPGLKPH (SEQ ID NO: 51)) were synthesized and coupled to keyhole limpet hemocyanin (Sigma Chemical Co. St. Louis, MO, H-2133) via a cysteine residue added to the N-terminus of the peptide using the heterobifunctional reagent, N-succinimidyl-3-(2-pyridyldithio)propionate (Pharmacia, Uppsala, Sweden). New Zealand White rabbits were immunized with 2 mg peptide conjugate initially in 50% (v/v) Freund's complete adjuvant and in 50% (v/v) Freund's incomplete adjuvant for subsequent immunizations. Rabbits were boosted fortnightly with 2 mg peptide conjugate and bled 7 days after booster injections. Anti-AMPK α₁ and α₂ peptide antibodies were purified by peptide affinity chromatography.

### Western Blotting

Multiple rat tissue western blots were prepared as follows. Rat tissues were homogenized in AMPK HB and a 2.5 - 7% polyethylene glycol 6000 fraction was prepared. The resultant pellet was resuspended in 5 ml HB and assayed for protein concentration. One hundred micrograms of each tissue fraction was analyzed by SDS PAGE (13% acrylamide gels); transferred to nitrocellulose (Schleicher & Schuell, Dassal, Germany); and probed with 3 µg/ml and 6 µg/ml affinity purified AMPK α₁ and α₂ antibodies, respectively. Primary antibody was detected using anti-rabbit IgG antibody conjugated to horseradish peroxidase (DAKO, Carpinteria, CA, USA) and 0.032% 3,3' -diamino-benzidine (D-5637, Sigma) together with 0.064% H₂O₂.

### Purification of AMPK α₂

Affinity purified AMPK α₂ antibody (2 mg) was coupled to CNBr-activated Sepharose 4B (Pharmacia, Uppsala, Sweden) according to the manufacturer's instructions. The unbound fraction from the substrate affinity column was applied directly to the AMPK α₂ antibody column, washed with 5 volumes of PBS and eluted with 200 mM glycine buffer pH 2.5 and immediately neutralized.

### EXAMPLE 3: Isolation of cDNAs Encoding AMPK Non-Catalytic Subunits

### AMPK isolation and peptide sequencing

Porcine and rat liver AMPK was isolated. Peptide sequences derived from the rat liver beta (40 kDa) and gamma (38 kDa) subunits were obtained after subunit separation by SDS gel electrophoresis, band elution and *in situ* protease digestion in accordance with procedures described by Mitchelhill et al. (1994) *J. Biol. Chem.* 269, 2361-2364 and Stapleton et al. (1994) *J. Biol. Chem.* 269, 29343-29346.

### AMPK β subuni t cDNA isolation

Peptide sequences derived from the AMPK β subunit were used to generate partial length AMPK β subunit cDNAs by the polymerase chain reaction (PCR) in accordance with procedures described by Gao et al. (1995) *Biochem. Biophys. Acta.* 1200, 73-82. One product, a 309 bp cDNA, was used to screen a rat liver λZAPII cDNA library (Stratagene). Filters were hybridized with ³²P-cDNA, labelled with alpha-³²P-CTP (3000mCi/mmol, New England Nuclear) by random priming (Random Primer cDNA Labeling System, Gibco/BRL), in 50% formamide, 10X Denhardt's, 1M NaCl, 50 mM Tris-Cl (pH 7.5), and 100 µg/ml salmon sperm DNA at 42°C for 18 hours. They were then washed at room temperature 3 x 10 minutes and then at 55°C for 15 minutes. Autoradiography was for overnight at -80°C. All plates were lifted in duplicate and positive plaques were purified through 3 additional rounds of plating and re-screening.

### AMPK γ subunit cDNA isolation

Where peptide sequences are listed herein, the letters Y,H,N and R indicate regions of degeneracy. For the AMPK γ subunit, a 67 bp cDNA was generated by the MOPAC technique described by Lee, C.C. and Caskey, C.T., (1990) in *PCR* Protocols, (Innis, M.A. Gelfand, D.H., Srinsky, J.J., and White, T.J. editors), pp. 46-53, Academic Press, Inc., London. Degenerate PCR primers were synthesized corresponding to the N-and C- terminal sequences of a 17-amino acid rat liver AMPK γ peptide (WDIYSKFDVINLAAEK (SEQ ID NO: 52). The sequence of the sense primer was GCGGATCCGTNGAYATHTA (SEQ ID NO: 53) and the sequence of the antisense primer was CGGAATTCYTTYTCNGCNGCNA (SEQ ID NO: 54). BamHI and EcoRI sites were added to the 5'-ends of these primers. The strategy was to create a non-degenerate nucleotide sequence corresponding to the middle portion of the peptide sequence that would be used in library screening. Total rat liver cDNA, prepared with oligo-dT and random hexamers (GIBCO/BRL pre-amplification kit), was used with PCR to amplify a 67-mer (including primers) oligonucleotide corresponding to a portion of the AMPK γ cDNA. The purified PCR product was digested with BamHI and EcoRI and ligated into pBluescript plasmid for transformation of DH5α bacteria. Colony hybridization was employed to identify clones of interest; colonies were lifted from replica plates onto nitrocellulose filters. Following bacterial lysis and DNA denaturation, filters were probed with a mixture of two ³²P-end-labeled degenerate oligonucleotide probes corresponding to amino acid sequence (KFDVINLA (SEQ ID NO: 55)) internal to that of the two PCR primers. These oligonucleotides (#1: AARTTYGAYGTNATHAAYCTNGC (SEQ ID NO: 56); #2: AARTTYGAYGTNATHAAYTTRGC SEQ ID NO: 57)) were added in a ratio of two parts oligo #1 to one part oligo #2 to reflect the degeneracy of the Leu codon. Positive colonies were identified and plasmid DNA isolated from each for sequence analysis. One such cDNA was chosen and the non-degenerate "core" 23-mer oligonucleotide sequence was then synthesized for use in library screening (CTCCAAGTTTGATGTTATCAACC (SEQ ID NO: 58)). Screening of approximately 10⁶ plaques with this probe, however, did not yield any positive clones.

The non-degenerate 23-mer cDNA was then used in conjugation with degenerate primers constructed from two other peptide sequences to generate a larger AMPK γ cDNA by PCR. Both sense and antisense degenerate oligonucleotide primers corresponding to the peptide sequences, EELQIG (SEQ ID NO: 59) and FPKPEFM (SEQ ID NO: 60), were used together with the sense MOPAC-derived non-degenerate sequence to generate all possible PCR products, using rat liver cDNA as template. The largest product (192 bp) obtained was subcloned in pCR-Script (Stratagene) and sequenced. This sequence, which actually had a predicted amino acid sequence corresponding to all three AMPK γ peptides used in the PCR strategy, was then used for library screening, as above. Screening of 2 x 10⁶ plaques with this larger PCR product yielded several positive clones; however, none of the rat cDNAs (1-1.3 kb) isolated corresponded to a full-length open reading frame. In an effort to extend the sequence to the 5'-end of the ORF, a primer extension library was constructed using a AMPK γ-specific antisense primer (Stratagene; λZAPII). Additional screening of this library, while yielding some 5'-extended sequence, did not yield the start Met codon. The application of a 5'-RACE strategy with rat liver cDNA was also unsuccessful in attempts at sequence extension, although a 5'-RACE product from porcine liver was obtained. The most 5' rat cDNA sequence (520 bp) was then used to screen a human fetal liver library, which yielded a full-length AMPK γ cDNA.

### Plasmid Preparation and DNA sequencing

Plasmid DNA was prepared using Qiagen Mini- or Midi- columns (Chatsworth, CA) according to the manufacturer's instructions. DNA was sequenced, with vector or gene-specific primers, using an Applied Biosystems Prism(tm) (Foster City, CA) ready reaction Dye Deoxy Terminator Cycle Sequencing kit, and cycled in a Perkin-Elmer PCR Thermocycler, according to the manufacturers' instructions. Dye terminators were removed from the resulting sequence reactions using a Centri-Step column (Princeton Separations, Inc.). The purified sequencing reactions were then dried in a Speed-Vac and analyzed on an automated DNA sequencer (Applied Biosystems Model 373).

### Bacterial Expression of cDNAs

Full-length rat AMPK β subunit cDNA and a partial length rat AMPK γ (aa 33-331) subunit cDNA were expressed in *E. coli* using the pET vector system, which introduces polyhistidine (His6) and T7 fusion epitope tag sequences (Novagen, Madison, WI). Bacterial expression was induced with 1.0 mM IPTG at 37°C for 2 hours. Expressed protein was detected by both Coomassie blue staining and immunoblotting with anti-T7 monoclonal antibody (Novagen). The fusion proteins were purified from the inclusion bodies of bacteria by nickel affinity chromatography under denaturing conditions. His6-AMPK β or His6-AMPK γ were solubilized from the inclusion bodies in 6 M urea, according to manufacturer's instructions. After sample application, the column was washed extensively with Tris-Cl (20 mM; pH 7.9), 0.5 M NaCl (0.5 M), imidazole (20 mM) and urea (6 M). The His6-protein was eluted with the same buffer containing 300 mM imidazole.

### Cellular expression of cDNAs

Full-length rat AMPK β cDNA, a partial length rat AMPK γ (aa 33-331) and full-length human AMPK γ subunit cDNAs were also expressed in COS7 cells. cDNAs were cloned into a pMT2 vector in-frame with a hemagglutinin (HA) epitope tag (pMT2-HA). Transfection was done using Lipofectamine reagent (Gibco/BRL), according to the manufacturer's general protocol. Cells were plated at 3 x 10⁵/well in 6 well plates in DMEM containing 10% fetal calf serum and penicillin/streptomycin. The following day, the cells were switched to serum-free, antibiotic-free DMEM and then lipofectamine-DNA conjugates (2 µg of DNA; 10 µl lipofectamine per well) diluted in the same medium were added. After 5 hours incubation at 37°C, an equal volume of medium containing 20% fetal calf serum was added to each well. The following morning, the medium was switched to the original cell medium. Cells were harvested 48 hours after transfection. After washing with PBS, cells were lysed in a buffer containing Tris-Cl (50 mM; pH 7.5), NaCl (100 mM), NaF (50 mM), NaPPᵢ (5 mM), EDTA (1 mM), DTT (2 mM) and NP-40 (0.5%) with several protease inhibitors.

For complete lysis, cells were placed on ice for 15 minutes followed by scraping and vigorous vortexing (15 seconds) of the lysate. After clearing of debris by brief centrifugation, this lysate was used for SDS gel electrophoresis and immunoblotting. Blots were probed with an anti-HA monoclonal antibody (derived from the 12CA5 hybridoma line). After secondary probing with an anti-mouse IgG-peroxidase antibody, blots were developed by ECL (Amersham).

### Northern Blot Analysis

Total RNA was isolated from the tissues of male Sprague-Dawley rats (150-200 grams body weight; Charles River) or from the lactating mammary gland of female rats using a guanidium isothiocyanate-lithium chloride method. RNAs were fractionated on 1% agarose/formaldehyde gels with capillary transfer to nitrocellulose (MSI). cDNA probes were labelled by random priming.

Hybridization was carried in 5x Denhardt' s, 0.2 M Tris (pH 7.4), 1M NaCl and 0.1 mg/ml salmon sperm DNA at 42°C for 20 hours. Filters were washed sequentially with 2X SSPE/0.1% SDS (room temperature; 2 x 15 minutes), 0.2 X SSPE/0.1% SDS (room temperature; 2 x 15 minutes) and with 0.2X SSPE/0.1% SDS (55°C; 2 x 15 minutes). Autoradiography on Kodak XAR film with enhancing screens was at -80°C for 18-48 hours.

### DNA Sequence Analysis and DNA sequences

DNA sequences were analyzed using MacVector (r) and the GCG software package. Sequences were compared to the data base using BLAST and GCG; amino acid alignments were made using the Pileup program of GCG. Sequences were formatted using an Excel(r) macro. The DNA sequences described herein have been deposited in the GenBank with the following accession numbers: rat liver AMPK β (U42411), rat liver AMPK γ (U42413) and human fetal liver AMPK γ (U42412).

## Claims

1. A nucleic acid sequence encoding mammalian AMPK α₁, the nucleic acid sequence comprising SEQ ID NO:44.

2. A vector comprising a nucleic acid sequence of claim 1.

3. A host cell comprising a vector of claim 2.

4. A recombinant polypeptide encoded by the nucleic acid sequence of claim 1.

5. A method of producing mammalian AMPK α₁ comprising:
(a) culturing cells of claim 3 under conditions which allow expression of the nucleic acid sequence encoding mammalian AMPK α₁; and
(b) recovering the expressed AMPK α₁ from the cell.

6. An oligonucleotide probe comprising at least 10 nucleotides, said oligonucleotide probe being capable of hybridizing to a nucleic acid sequence of claim 1, having SEQ ID NO: 67, which encodes amino acids 352-366 of AMPK α₁, but not to a nucleic acid sequencing encoding a mammalian AMPK α₂ polypeptide encoded by nucleic acid molecules having sequences of SEQ ID NO: 65 or 66.

7. A purified polypeptide or biologically active fragment thereof encoded by a nucleic acid sequence of claim 1, wherein, said biologically active fragment is not present in a mammalian AMPK α₂ polypeptide encoded by nucleic acid molecules having sequences of SEQ ID NO: 65 or 66 and comprises:
(a) at least a portion of SEQ ID NOs: 1-12, 15-32, 35-38, 40 or 43
(b) SEQ ID NOs: 14, 34, 39, 41 or 42;
(c) retains the ability to stimulate phosphorylation of protein molecules; or
(d) retains the ability to mimic the binding of native AMPK α₁ polypeptide to at least one antibody or substrate molecule.

## Patentansprüche

1. Nukleinsäuresequenz, die Säugetier-AMPK-α₁ kodiert, wobei die Nukleinsäuresequenz SEQ ID-NR. 44 umfasst.

2. Vektor, der eine Nukleinsäuresequenz aus Anspruch 1 umfasst.

3. Wirtszelle, die einen Vektor aus Anspruch 2 umfasst.

4. Rekombinantes Polypeptid, das durch die Nukleinsäuresequenz aus Anspruch 1 kodiert wird.

5. Verfahren zur Herstellung von Säugetier-AMPK-α₁, umfassend:
(a) Züchten der Zellen aus Anspruch 3, unter Bedingungen, die das Exprimieren der Nukleinsäuresequenz, die Säugetier-AMPK-α₁ kodiert, ermöglichen; und
(b) Gewinnung von exprimiertem AMKP-α₁ aus der Zelle

6. Oligonukleotidsonde, umfassend mindestens 10 Nukleotide, wobei diese Oligonukleotidsonde in der Lage ist, mit einer Nukleinsäursequenz aus Anspruch 1 mit der SEQ ID-NR. 67 zu hybridisieren, welche die Aminosäuren 352-366 von AMPK-α₁ kodiert, aber nicht mit einer Nukleinsäuresequenz zu hybridisieren, die ein Säugetier-AMPK-α₂-Polypeptid kodiert, das durch Nukleinsäuremoleküle mit den Sequenzen SEQ ID-NR. 65 oder 66 kodiert wird.

7. Gereinigtes Polypeptid oder dessen biologisch aktives Fragment, das von einer Nukleinsäuresequenz aus Anspruch 1 kodiert wird, wobei das biologisch aktive Fragment nicht in einem Säugetier-AMPK-α-Potypeptid vorliegt, das von Nukleinsäuremolekülen mit Sequenzen der SEQ ID-NRn. 65 oder 66 kodiert wird, und umfasst:
(a) mindestens einen Teil der SEQ ID-NRn. 1-12, 15-32, 35-38, 40 oder 43
(b) SEQ ID-Nm. 14, 34, 39, 41 oder 42;
(c) Beibehalten der Fähigkeit, die Phosphorylierung von Proteinmolekülen zu stimulieren; oder
(d) Beibehalten der Fähigkeit, die Bindung von natürlichem AMPK-α₁-Polypeptid an mindestens einen Antikörper oder an ein Substratmolekül nachzuahmen.

## Revendications

1. Séquence d'acide nucléique codant pour l'AMPK α₁ de mammifère, la séquence d'acide nucléique comprenant SEQ ID NO: 44.

2. Vecteur comprenant une séquence d'acide nucléique de la revendication 1.

3. Cellule hôte comprenant un vecteur de la revendication 2.

4. Polypeptide recombinant codé par la séquence d'acide nucléique de la revendication 1.

5. Procédé dé production d'AMPK α₁ de mammifère, comprenant :
(a) la culture de cellules de la revendication 3 dans des conditions qui permettent une expression de la séquence d'acide nucléique codant pour l'AMPK α₁ de mammifère ; et
(b) la récupération de l'AMPK α₁ exprimé à partir de la cellule.

6. Sonde oligonucléotidique comprenant au moins 10 nucléotides, ladite sonde oligonucléotidique étant capable de s'hybrider à une séquence d'acide nucléique de la revendication 1, ayant SEQ ID NO: 67, qui code pour les acides aminés 352-366 d'AMPK α₁, mais non à une séquence d'acide nucléique codant pour un polypeptide AMPK α₂ de mammifère codé par des molécules d'acide nucléique ayant des séquences de SEQ ID NO: 65 ou 66.

7. Polypeptide purifié ou fragment biologiquement actif de celui-ci codé par une séquence d'acide nucléique de la revendication 1, dans lequel ledit fragment biologiquement actif n'est pas présent dans un polypeptide AMPK α₂ de mammifère codé par des molécules d'acide nucléique ayant des séquences de SEQ ID NO: 65 ou 66 et comprend :
(a) au moins une partie de SEQ ID Nos: 1-12, 15-32, 35-38, 40 ou 43 ;
(b) SEQ ID Nos: 14, 34, 39, 41 ou 42 ;
(c) conserve l'aptitude à stimuler la phosphcrylation de molécules de protéine ; ou
(d) conserve l'aptitude à mimer la liaison de polypeptide AMPK α₁ natif à au moins une molécule d'anticorps ou de substrat.


### SEQUENCE LISTING

(1) GENERAL INFORMATION:
   (i) APPLICANT: Dartmouth College, St. Vincents Institute of Medical Research, Kemp et al.
   (ii) TITLE OF INVENTION: Novel AMP Activated Protein Kinase
   (iii) NUMBER OF SEQUENCES: 64
   (iv) CORRESPONDENCE ADDRESS:
      (A) ADDRESSEE: Jane Massey Licata, Esq.
      (B) STREET: 210 Lake Drive East, Suite 201
      (C) CITY: Cherry Hill
      (D) STATE: NJ
      (E) COUNTRY: USA
      (F) ZIP: 08002
   (v) COMPUTER READABLE FORM:
      (A) MEDIUM TYPE: DISKETTE, 3.5 INCH, 1.44 Mb STORAGE
      (B) COMPUTER: IBM 486
      (C) OPERATING SYSTEM: WINDOWS FOR WORKGROUPS
      (D) SOFTWARE: WORDPERFECT 5.1
   (vi) CURRENT APPLICATION DATA:
      (A) APPLICATION NUMBER: PCT/US97/00270
      (B) FILING DATE: January 7, 1997
      (C) CLASSIFICATION:
   (vii) PRIOR APPLICATION DATA:
      (A) APPLICATION NUMBER: PN7450
      (B) FILING DATE: 8 JAN 1996
   (viii) ATTORNEY/AGENT INFORMATION:
      (A) NAME: Jane Massey Licata
      (B) REGISTRATION NUMBER: 32,257
      (C) REFERENCE/DOCKET NUMBER: DC-0028
   (ix) TELECOMMUNICATION INFORMATION:
      (A) TELEPHONE: (609) 779-2400
      (B) TELEFAX: (609) 779-8488
(2) INFORMATION FOR SEQ ID NO: 1:
   (i) SEQUENCE CHARACTERISTICS:
      (A) LENGTH: 347
      (B) TYPE: Amino acid
      (D) TOPOLOGY: Linear
   (xi) SEQUENCE DESCRIPTION: SEQ ID NO: 1:
   Met Ala Glu Lys Gln Lys His Asp Gly Arg Val Lys Ile Gly His
   1               5                   10                  15
   Tyr Ile Leu Gly Asp Thr Leu Gly Val Gly Thr Phe Gly Lys Val
                   20                  25                  30
   Lys Val Gly Lys His Glu Leu Thr Gly His Lys Val Ala Val Lys
                   35                  40                  45
   Ile Leu Asn Arg Gln Lys Ile Arg Ser Leu Asp Val Val Gly Lys
                   50                  55                  60
   Ile Arg Arg Glu Ile Gln Asn Leu Lys Leu Phe Arg His Pro His
                   65                  70                  75
   Ile Ile Lys Leu Tyr Gln Val Ile Ser Thr Pro Ser Asp Ile Phe
                   80                  85                  90
   Met Val Met Glu Tyr Val Ser Gly Gly Glu Leu Phe Asp Tyr Ile
                   95                  100                 105
   Cys Lys Asn Gly Arg Leu Asp Glu Lys Glu Ser Arg Arg Leu Phe
                   110                 115                 120
   Gln Gln Ile Leu Ser Gly Val Asp Tyr Cys His Arg His Met Val
                   125                 130                 135
   Val His Arg Asp Leu Lys Pro Glu Asn Val Leu Leu Asp Ala His
                   140                 145                 150
   Met Asn Ala Lys Ile Ala Asp Phe Gly Leu Ser Asn Met Met Ser
                   155                 160                 165
   Asp Gly Glu Phe Leu Arg Thr Ser Cys Gly Ser Pro Asn Tyr Ala
                   170                 175                 180
   Ala Pro Glu Val Ile Ser Gly Arg Leu Tyr Ala Gly Pro Glu Val
                   185                 190                 195
   Asp Ile Trp Ser Ser Gly Val Ile Leu Tyr Ala Leu Leu Cys Gly
                   200                 205                 210
   Thr Leu Pro Phe Asp Asp Asp His Val Pro Thr Leu Phe Lys Lys
                   215                 220                 225
   Ile Cys Asp Gly Ile Phe Tyr Thr Pro Gln Tyr Leu Asn Pro Ser
                   230                 235                 240
   Val Ile Ser Leu Leu Lys His Met Leu Gln Val Asp Pro Met Lys
                   245                 250                 255
   Arg Ala Thr Ile Lys Asp Ile Arg Glu His Glu Trp Phe Lys Gln
                   260                 265                 270
   Asp Leu Pro Lys Tyr Leu Phe Pro Glu Asp Pro Ser Tyr Ser Ser
                   275                 280                 285
   Thr Met Ile Asp Asp Glu Ala Leu Lys Glu Val Cys Glu Lys Phe
                   290                 295                 300
   Glu Cys Ser Glu Glu Glu Val Leu Ser Cys Leu Tyr Asn Arg Asn
                   305                 310                 315
   His Gln Asp Pro Leu Ala Val Ala Tyr His Leu Ile Ile Asp Asn
                   320                 325                 330
   Arg Arg Ile Met Asn Glu Ala Lys Asp Phe Tyr Leu Ala Thr Ser
                   335                 340                 345
   Pro Pro
(2) INFORMATION FOR SEQ ID NO: 2:
   (i) SEQUENCE CHARACTERISTICS:
      (A) LENGTH: 11
      (B) TYPE: Amino acid
      (D) TOPOLOGY: Linear
   (xi) SEQUENCE DESCRIPTION: SEQ ID NO: 2:
   Asp Ser Phe Leu Asp Asp His His Leu Thr Arg
   1               5                   10
(2) INFORMATION FOR SEQ ID NO: 3:
   (i) SEQUENCE CHARACTERISTICS:
      (A) LENGTH: 56
      (B) TYPE: Amino acid
      (D) TOPOLOGY: Linear
   (xi) SEQUENCE DESCRIPTION: SEQ ID NO: 3:
   Pro His Pro Glu Arg Val Pro Phe Leu Val Ala Glu Thr Pro Arg
   1               5                   10                  15
   Ala Arg His Thr Leu Asp Glu Leu Asn Pro Gln Lys Ser Lys His
                   20                  25                  30
   Gln Gly Val Arg Lys Ala Lys Trp His Leu Gly Ile Arg Ser Gln
                   35                  40                  45
   Ser Arg Pro Asn Asp Ile Met Ala Glu Val Cys
                   50                  55
(2) INFORMATION FOR SEQ ID NO: 4:
   (i) SEQUENCE CHARACTERISTICS:
      (A) LENGTH: 70
      (B) TYPE: Amino acid
      (D) TOPOLOGY: Linear
   (xi) SEQUENCE DESCRIPTION: SEQ ID NO: 4:
(2) INFORMATION FOR SEQ ID NO: 5:
   (i) SEQUENCE CHARACTERISTICS:
      (A) LENGTH: 64
      (B) TYPE: Amino acid
      (D) TOPOLOGY: Linear
   (xi) SEQUENCE DESCRIPTION: SEQ ID NO: 5:
   Ser Ile Ser Asn Tyr Arg Ser Cys Gln Arg Ser Asp Ser Asp Ala
   1               5                   10                  15
   Glu Ala Gln Gly Lys Pro Ser Glu Val Ser Leu Thr Ser Ser Val
                   20                  25                  30
   Thr Ser Leu Asp Ser Ser Pro Val Asp Val Ala Pro Arg Pro Gly
                   35                  40                  45
   Ser His Thr Ile Glu Phe Phe Glu Met Cys Ala Asn Leu Ile Lys
                   50                  55                  60
   Ile Leu Ala Gln
(2) INFORMATION FOR SEQ ID NO: 6:
   (i) SEQUENCE CHARACTERISTICS:
      (A) LENGTH: 257
      (B) TYPE: Amino acid
      (D) TOPOLOGY: Linear
   (xi) SEQUENCE DESCRIPTION: SEQ ID NO: 6:
   Gly His Tyr Ile Leu Gly Asp Thr Leu Gly Val Gly Thr Phe Gly
   1               5                   10                  15
   Lys Val Lys Val Gly Lys His Glu Leu Thr Gly His Lys Val Ala
                   20                  25                  30
   Val Lys Ile Leu Asn Arg Gln Lys Ile Arg Ser Leu Asp Val Val
                   35                  40                  45
   Gly Lys Ile Arg Arg Glu Ile Gln Asn Leu Lys Leu Phe Arg His
                   50                  55                  60
   Pro His Ile Ile Lys Leu Tyr Gln Val Ile Ser Thr Pro Ser Asp
                   65                  70                  75
   Ile Phe Met Val Met Glu Tyr Val Ser Gly Gly Glu Leu Phe Asp
                   80                  85                  90
   Tyr Ile Cys Lys Asn Gly Arg Leu Asp Glu Lys Glu Ser Arg Arg
                   95                  100                 105
   Leu Phe Gln Gln Ile Leu Ser Gly Val Asp Tyr Cys His Arg His
                   110                 115                 120
   Met Val Val His Arg Asp Leu Lys Pro Glu Asn Val Leu Leu Asp
                   125                 130                 135
   Ala His Met Asn Ala Lys Ile Ala Asp Phe Gly Leu Ser Asn Met
                   140                 145                 150
   Met Ser Asp Gly Glu Phe Leu Arg Thr Ser Cys Gly Ser Pro Asn
                   155                 160                 165
   Tyr Ala Ala Pro Glu Val Ile Ser Gly Arg Leu Tyr Ala Gly Pro
                   170                 175                 180
   Glu Val Asp Ile Trp Ser Ser Gly Val Ile Leu Tyr Ala Leu Leu
                   185                 190                 195
   Cys Gly Thr Leu Pro Phe Asp Asp Asp His Val Pro Thr Leu Phe
                   200                 205                 210
   Lys Lys Ile Cys Asp Gly Ile Phe Tyr Thr Pro Gln Tyr Leu Asn
                   215                 220                 225
   Pro Ser Val Ile Ser Leu Leu Lys His Met Leu Gln Val Asp Pro
                   230                 235                 240
   Met Lys Arg Ala Thr Ile Lys Asp Ile Arg Glu His Glu Trp Phe
                   245                 250                 255
   Lys Gln
(2) INFORMATION FOR SEQ ID NO: 7:
   (i) SEQUENCE CHARACTERISTICS:
      (A) LENGTH: 55
      (B) TYPE: Amino acid
      (D) TOPOLOGY: Linear
   (xi) SEQUENCE DESCRIPTION: SEQ ID NO: 7:
   Glu Ala Leu Lys Glu Val Cys Glu Lys Phe Glu Cys Ser Glu Glu
   1               5                   10                  15
   Glu Val Leu Ser Cys Leu Tyr Asn Arg Asn His Gln Asp Pro Leu
                   20                  25                  30
   Ala Val Ala Tyr His Leu Ile Ile Asp Asn Arg Arg Ile Met Asn
                   35                  40                  45
   Glu Ala Lys Asp Phe Tyr Leu Ala Thr Ser
                   50                  55
(2) INFORMATION FOR SEQ ID NO: 8:
   (i) SEQUENCE CHARACTERISTICS:
      (A) LENGTH: 9
      (B) TYPE: Amino acid
      (D) TOPOLOGY: Linear
   (xi) SEQUENCE DESCRIPTION: SEQ ID NO: 8:
   Phe Leu Asp Asp His His Leu Thr Arg
   1               5
(2) INFORMATION FOR SEQ ID NO: 9:
   (i) SEQUENCE CHARACTERISTICS:
      (A) LENGTH: 56
      (B) TYPE: Amino acid
      (D) TOPOLOGY: Linear
   (xi) SEQUENCE DESCRIPTION: SEQ ID NO: 9:
   Pro His Pro Glu Arg Val Pro Phe Leu Val Ala Glu Thr Pro Arg
   1               5                   10                  15
   Ala Arg His Thr Leu Asp Glu Leu Asn Pro Gln Lys Ser Lys His
                   20                  25                  30
   Gln Gly Val Arg Lys Ala Lys Trp His Leu Gly Ile Arg Ser Gln
                   35                  40                  45
   Ser Arg Pro Asn Asp Ile Met Ala Glu Val Cys
                   50                  55
(2) INFORMATION FOR SEQ ID NO: 10:
   (i) SEQUENCE CHARACTERISTICS:
      (A) LENGTH: 10
      (B) TYPE: Amino acid
      (D) TOPOLOGY: Linear
   (xi) SEQUENCE DESCRIPTION: SEQ ID NO: 10:
   Arg Pro Asn Asp Ile Met Ala Glu Val Cys
   1               5                   10
(2) INFORMATION FOR SEQ ID NO: 11:
   (i) SEQUENCE CHARACTERISTICS:
      (A) LENGTH: 70
      (B) TYPE: Amino acid
      (D) TOPOLOGY: Linear
   (xi) SEQUENCE DESCRIPTION: SEQ ID NO: 11:
   Arg Ala Ile Lys Gln Leu Asp Tyr Glu Trp Lys Val Val Asn Pro
   1               5                   10                  15
   Tyr Tyr Leu Arg Val Arg Arg Lys Asn Pro Val Thr Ser Thr Tyr
                   20                  25                  30
   Ser Lys Met Ser Leu Gln Leu Tyr Gln Val Asp Ser Arg Thr Tyr
                   35                  40                  45
   Leu Leu Asp Phe Arg Ser Ile Asp Asp Glu Ile Thr Glu Ala Lys
                   50                  55                  60
   Ser Gly Thr Ala Thr Pro Gln Arg Ser Gly
                   65                  70
(2) INFORMATION FOR SEQ ID NO: 12:
   (i) SEQUENCE CHARACTERISTICS:
      (A) LENGTH: 64
      (B) TYPE: Amino acid
      (D) TOPOLOGY: Linear
   (xi) SEQUENCE DESCRIPTION: SEQ ID NO: 12:
   Ser Val Ser Asn Tyr Arg Ser Cys Gln Arg Ser Asp Ser Asp Ala
   1               5                   10                  15
   Glu Ala Gln Gly Lys Ser Ser Glu Val Ser Leu Thr Ser Ser Val
                   20                  25                  30
   Thr Ser Leu Asp Ser Ser Pro Val Asp Leu Thr Pro Arg Pro Gly
                   35                  40                  45
   Ser His Thr Ile Glu Phe Phe Glu Met Cys Ala Asn Leu Ile Lys
                   50                  55                  60
   Ile Leu Ala Gln
(2) INFORMATION FOR SEQ ID NO: 13:
   (i) SEQUENCE CHARACTERISTICS:
      (A) LENGTH: 22
      (B) TYPE: Amino acid
      (D) TOPOLOGY: Linear
   (xi) SEQUENCE DESCRIPTION: SEQ ID NO: 13:
   Asp Gly Arg Val Lys Ile Gly His Tyr Ile Leu Gly Asp Thr Leu
   1               5                   10                  15
   Gly Val Gly Thr Phe Gly Lys
                   20
(2) INFORMATION FOR SEQ ID NO: 14:
   (i) SEQUENCE CHARACTERISTICS:
      (A) LENGTH: 16
      (B) TYPE: Amino acid
      (D) TOPOLOGY: Linear
   (xi) SEQUENCE DESCRIPTION: SEQ ID NO: 14:
   Asp Glu Lys Glu Ser Arg Arg Leu Phe Gln Gln Ile Leu Ser Gly
   1               5                   10                  15
   Val
(2) INFORMATION FOR SEQ ID NO: 15:
   (i) SEQUENCE CHARACTERISTICS:
      (A) LENGTH: 33
      (B) TYPE: Amino acid
      (D) TOPOLOGY: Linear
   (xi) SEQUENCE DESCRIPTION: SEQ ID NO: 15:
   Asp Leu Lys Pro Glu Asn Val Leu Leu Asp Ala His Met Asn Ala
   1               5                   10                  15
   Lys Ile Ala Asp Phe Gly Leu Ser Asn Met Met Ser Asp Gly Glu
                   20                  25                  30
   Phe Leu Arg
(2) INFORMATION FOR SEQ ID NO: 16:
   (i) SEQUENCE CHARACTERISTICS:
      (A) LENGTH: 13
      (B) TYPE: Amino acid
      (D) TOPOLOGY: Linear
   (xi) SEQUENCE DESCRIPTION: SEQ ID NO: 16:
   Glu Val Ile Ser Gly Arg Leu Tyr Ala Gly Pro Glu Val
   1               5                   10
(2) INFORMATION FOR SEQ ID NO: 17:
   (i) SEQUENCE CHARACTERISTICS:
      (A) LENGTH: 9
      (B) TYPE: Amino acid
      (D) TOPOLOGY: Linear
   (xi) SEQUENCE DESCRIPTION: SEQ ID NO: 17:
   Xaa Met Leu Gln Val Asp Pro Met Lys
   1               5
(2) INFORMATION FOR SEQ ID NO: 18:
   (i) SEQUENCE CHARACTERISTICS:
      (A) LENGTH: 35
      (B) TYPE: Amino acid
      (D) TOPOLOGY: Linear
   (xi) SEQUENCE DESCRIPTION: SEQ ID NO: 18:
   Lys Asp Ile Arg Glu His Glu Xaa Phe Lys Gln Asp Leu Pro Lys
   1               5                   10                  15
   Tyr Leu Phe Pro Glu Asp Pro Ser Tyr Ser Xaa Thr Met Ile Asp
                   20                  25                  30
   Asp Glu Ala Leu Lys
                   35
(2) INFORMATION FOR SEQ ID NO: 19:
   (i) SEQUENCE CHARACTERISTICS:
      (A) LENGTH: 17
      (B) TYPE: Amino acid
      (D) TOPOLOGY: Linear
   (xi) SEQUENCE DESCRIPTION: SEQ ID NO: 19:
   Xaa Xaa Gln Asp Pro Leu Ala Val Ala Tyr His Leu Ile Ile Asp
   1               5                   10                  15
   Asn Arg
(2) INFORMATION FOR SEQ ID NO: 20:
   (i) SEQUENCE CHARACTERISTICS:
      (A) LENGTH: 9
      (B) TYPE: Amino acid
      (D) TOPOLOGY: Linear
   (xi) SEQUENCE DESCRIPTION: SEQ ID NO: 20:
   Asp Phe Tyr Leu Ala Thr Ser Pro Pro
   1               5
(2) INFORMATION FOR SEQ ID NO: 21:
   (i) SEQUENCE CHARACTERISTICS:
      (A) LENGTH: 11
      (B) TYPE: Amino acid
      (D) TOPOLOGY: Linear
   (xi) SEQUENCE DESCRIPTION: SEQ ID NO: 21:
   Asp Ser Phe Leu Asp Asp His His Leu Thr Arg
   1               5                   10
(2) INFORMATION FOR SEQ ID NO: 22:
   (i) SEQUENCE CHARACTERISTICS:
      (A) LENGTH: 10
      (B) TYPE: Amino acid
      (D) TOPOLOGY: Linear
   (xi) SEQUENCE DESCRIPTION: SEQ ID NO: 22:
   Val Pro Phe Leu Val Ala Glu Thr Pro Arg
   1               5                   10
(2) INFORMATION FOR SEQ ID NO: 23:
   (i) SEQUENCE CHARACTERISTICS:
      (A) LENGTH: 23
      (B) TYPE: Amino acid
      (D) TOPOLOGY: Linear
   (xi) SEQUENCE DESCRIPTION: SEQ ID NO: 23:
   Asp Glu Leu Asn Pro Gln Lys Xaa Lys His Gln Gly Val Arg Lys
   1               5                   10                  15
   Ala Lys Xaa His Leu Gly Ile Arg
                   20
(2) INFORMATION FOR SEQ ID NO: 24:
   (i) SEQUENCE CHARACTERISTICS:
      (A) LENGTH: 19
      (B) TYPE: Amino acid
      (D) TOPOLOGY: Linear
   (xi) SEQUENCE DESCRIPTION: SEQ ID NO: 24:
   Gln Leu Asp Tyr Glu Xaa Lys Val Val Asn Pro Tyr Tyr Leu Arg
   1               5                   10                  15
   VAL ARG ARG LYS
(2) INFORMATION FOR SEQ ID NO: 25:
   (i) SEQUENCE CHARACTERISTICS:
      (A) LENGTH: 25
      (B) TYPE: Amino acid
      (D) TOPOLOGY: Linear
   (xi) SEQUENCE DESCRIPTION: SEQ ID NO: 25:
   Lys Met Ser Leu Gln Leu Tyr Gln Val Asp Ser Arg Thr Tyr Leu
   1               5                   10                  15
   Leu Asp Phe Arg Ser Ile Asp Asp Xaa Ile
                   20                  25
(2) INFORMATION FOR SEQ ID NO: 26:
   (i) SEQUENCE CHARACTERISTICS:
      (A) LENGTH: 18
      (B) TYPE: Amino acid
      (D) TOPOLOGY: Linear
   (xi) SEQUENCE DESCRIPTION: SEQ ID NO: 26:
   Asp Ala Glu Ala Gln Gly Lys Ser Ser Glu Ala Ser Leu Thr Xaa
   1               5                   10                  15
   Ser Val Thr
(2) INFORMATION FOR SEQ ID NO: 27:
   (i) SEQUENCE CHARACTERISTICS:
      (A) LENGTH: 17
      (B) TYPE: Amino acid
      (D) TOPOLOGY: Linear
   (xi) SEQUENCE DESCRIPTION: SEQ ID NO: 27:
   Ile Gly His Tyr Ile Leu Gly Asp Thr Leu Gly Val Gly Thr Phe
   1               5                   10                  15
   Gly Lys
(2) INFORMATION FOR SEQ ID NO: 28:
   (i) SEQUENCE CHARACTERISTICS:
      (A) LENGTH: 26
      (B) TYPE: Amino acid
      (D) TOPOLOGY: Linear
   (xi) SEQUENCE DESCRIPTION: SEQ ID NO: 28:
   Leu Tyr Gln Val Ile Ser Thr Pro Ser Asp Ile Phe Met Val Met
   1               5                   10                  15
   Glu Tyr Val Ser Gly Gly Glu Leu Phe Asp Tyr
                   20                  25
(2) INFORMATION FOR SEQ ID NO: 29:
   (i) SEQUENCE CHARACTERISTICS:
      (A) LENGTH: 12
      (B) TYPE: Amino acid
      (D) TOPOLOGY: Linear
   (xi) SEQUENCE DESCRIPTION: SEQ ID NO: 29:
   Arg Leu Phe Gln Gln Ile Leu Ser Gly Val Asp Tyr
   1 5 10
(2) INFORMATION FOR SEQ ID NO: 30:
   (i) SEQUENCE CHARACTERISTICS:
      (A) LENGTH: 11
      (B) TYPE: Amino acid
      (D) TOPOLOGY: Linear
   (xi) SEQUENCE DESCRIPTION: SEQ ID NO: 30:
   Asp Leu Lys Pro Glu Asn Val Leu Leu Asp Ala
   1               5                   10
(2) INFORMATION FOR SEQ ID NO: 31:
   (i) SEQUENCE CHARACTERISTICS:
      (A) LENGTH: 17
      (B) TYPE: Amino acid
      (D) TOPOLOGY: Linear
   (xi) SEQUENCE DESCRIPTION: SEQ ID NO: 31:
   Ile Ala Asp Phe Gly Leu Ser Asn Met Met Ser Asp Gly Glu Phe
   1               5                   10                  15
   Leu Arg
(2) INFORMATION FOR SEQ ID NO: 32:
   (i) SEQUENCE CHARACTERISTICS:
      (A) LENGTH: 22
      (B) TYPE: Amino acid
      (D) TOPOLOGY: Linear
   (xi) SEQUENCE DESCRIPTION: SEQ ID NO: 32:
   Lys Ile Xaa Asp Gly Ile Phe Tyr Thr Pro Gln Tyr Leu Asn Pro
   1               5                   10                  15
   Xaa Val Ile Xaa Leu Leu Lys
                   20
(2) INFORMATION FOR SEQ ID NO: 33:
   (i) SEQUENCE CHARACTERISTICS:
      (A) LENGTH: 5
      (B) TYPE: Amino acid
      (D) TOPOLOGY: Linear
   (xi) SEQUENCE DESCRIPTION: SEQ ID NO: 33:
   Asp Ile Arg Glu His
   1 5
(2) INFORMATION FOR SEQ ID NO: 34:
   (i) SEQUENCE CHARACTERISTICS:
      (A) LENGTH: 20
      (B) TYPE: Amino acid
      (D) TOPOLOGY: Linear
   (xi) SEQUENCE DESCRIPTION: SEQ ID NO: 34:
   Tyr Leu Phe Pro Glu Asp Pro Ser Tyr Ser Xaa Xaa Met Ile Asp
   1 5 10 15
   Asp Glu Ala Leu Lys
   20
(2) INFORMATION FOR SEQ ID NO: 35:
   (i) SEQUENCE CHARACTERISTICS:
      (A) LENGTH: 16
      (B) TYPE: Amino acid
      (D) TOPOLOGY: Linear
   (xi) SEQUENCE DESCRIPTION: SEQ ID NO: 35:
   Asn His Gln Asp Pro Leu Ala Val Ala Tyr His Leu Ile Ile Asp
   1 5 10 15
   Asn
(2) INFORMATION FOR SEQ ID NO: 36:
   (i) SEQUENCE CHARACTERISTICS:
      (A) LENGTH: 9
      (B) TYPE: Amino acid
      (D) TOPOLOGY: Linear
   (xi) SEQUENCE DESCRIPTION: SEQ ID NO: 36:
   Asp Phe Tyr Leu Ala Thr Xaa Pro Pro
   1 5
(2) INFORMATION FOR SEQ ID NO: 37:
   (i) SEQUENCE CHARACTERISTICS:
      (A) LENGTH: 9
      (B) TYPE: Amino acid
      (D) TOPOLOGY: Linear
   (xi) SEQUENCE DESCRIPTION: SEQ ID NO: 37:
   Asp Xaa Phe Leu Asp Asp His Xaa Leu
   1               5
(2) INFORMATION FOR SEQ ID NO: 38:
   (i) SEQUENCE CHARACTERISTICS:
      (A) LENGTH: 10
      (B) TYPE: Amino acid
      (D) TOPOLOGY: Linear
   (xi) SEQUENCE DESCRIPTION: SEQ ID NO: 38:
   Val Pro Phe Leu Val Ala Glu Thr Pro Arg
   1               5                   10
(2) INFORMATION FOR SEQ ID NO: 39:
   (i) SEQUENCE CHARACTERISTICS:
      (A) LENGTH: 5
      (B) TYPE: Amino acid
      (D) TOPOLOGY: Linear
   (xi) SEQUENCE DESCRIPTION: SEQ ID NO: 39:
   Trp His Leu Gly Ile
   1               5
(2) INFORMATION FOR SEQ ID NO: 40:
   (i) SEQUENCE CHARACTERISTICS:
      (A) LENGTH: 14
      (B) TYPE: Amino acid
      (D) TOPOLOGY: Linear
   (xi) SEQUENCE DESCRIPTION: SEQ ID NO: 40:
   Xaa Gln Ser Arg Pro Asn Asp Ile Met Ala Glu Val Xaa Arg
   1               5                   10
(2) INFORMATION FOR SEQ ID NO: 41:
   (i) SEQUENCE CHARACTERISTICS:
      (A) LENGTH: 10
      (B) TYPE: Amino acid
      (D) TOPOLOGY: Linear
   (xi) SEQUENCE DESCRIPTION: SEQ ID NO: 41:
   Val Val Asn Pro Tyr Tyr Leu Arg Val Arg
   1               5                   10
(2) INFORMATION FOR SEQ ID NO: 42:
   (i) SEQUENCE CHARACTERISTICS:
      (A) LENGTH: 18
      (B) TYPE: Amino acid
      (D) TOPOLOGY: Linear
   (xi) SEQUENCE DESCRIPTION: SEQ ID NO: 42:
   Met Ser Leu Gln Leu Tyr Gln Val Asp Ser Arg Thr Tyr Leu Leu
   1               5                   10                  15
   Leu Phe Arg
(2) INFORMATION FOR SEQ ID NO: 43:
   (i) SEQUENCE CHARACTERISTICS:
      (A) LENGTH: 12
      (B) TYPE: Amino acid
      (D) TOPOLOGY: Linear
   (xi) SEQUENCE DESCRIPTION: SEQ ID NO: 43:
   Xaa Asp Ser Asp Ala Glu Ala Gln Gly Lys Pro Ser
   1               5                   10
(2) INFORMATION FOR SEQ ID NO: 44:
   (i) SEQUENCE CHARACTERISTICS:
      (A) LENGTH: 1647
      (B) TYPE: Nucleic acid
      (C) STRANDEDNESS: Single
      (D) TOPOLOGY: Linear
   (iv) ANTI-SENSE: No
   (xi) SEQUENCE DESCRIPTION: SEQ ID NO: 44:
   ATGGCCGAGA AGCAGAAGCA CGACGGGCGG GTGAAGATCG GCCACTACAT 50
   CCTGGGGGAC ACGCTGGGCG TCGGCACCTT CGGGAAAGTG AAGGTGGGCA 100
   AGCACGAGTT GACTGGACAT AAAGTTGCTG TGAAGATACT CAACCGGCAG 150
   AAGATTCGAA GCCTGGACGT GGTCGGGAAA ATCCGCAGAG AGATCCAGAA 200
   CCTGAAGCTT TTCAGGCACC CTCATATAAT CAAACTGTAC CAGGTCATCA 250
   GTACACCGTC TGATATTTTC ATGGTCATGG AATATGTCTC AGGAGGAGAG 300
   CTATTTGATT ATATCTGTAA AAATGGAAGG TTGGACGAAA AGGAGAGTCG 350
   ACGTCTGTTC CAGCAGATCC TTTCTGGTGT GGACTATTGT CACAGGCATA 400
   TGGTGGTCCA CAGAGATTTG AAACCTGAAA ACGTCCTGCT TGATGCACAC 450
   ATGAATGCAA AGATAGCCGA CTTCGGTCTT TCAAACATGA TGTCAGATGG 500
   TGAATTTTTA AGAACGAGCT GTGGCTCGCC CAATTATGCT GCACCAGAAG 550
   TAATTTCAGG AAGATTCTAC GCAGGCCCTG AAGTAGACAT CTGGAGCAGC 600
   GGGGTCATTC TCTATGCTTT GCTGTGTGGA ACTCTCCCTT TTGATGATGA 650
   CCACGTGCCA ACTCTTTTTA AGAAGATATG TGACGGGATA TTTTATACCC 700
   CTCAGTATTT GAATCCCTCT GTAATAAGCC TTTTGAAGCA TATGCTGCAG 750
   GTAGATCCTA TGAAGAGGGC CACAATAAAA GATATCAGGG AACATGAATG 800
   GTTTAAGCAG GACCTTCCAA AATATCTCTT TCCTGAAGAC CCGTCTTATA 850
   GTTCAACCAT GATTGATGAT GAAGCCTTAA AAGAAGTGTG TGAGAAGTTC 900
   GAGTGCTCAG AGGAGGAGGT CCTCAGCTGC CTGTACAACA GAAACCACCA 950
   GGACCCACTG GCAGTTGCCT ACCACCTCAT AATAGACAAC AGGAGAATAA 1000
   TGAACGAAGC CAAAGATTTC TACTTGGCAA CAAGCCCACC CGATTCTTTC 1050
   CTCGATGATC ACCATTTAAC TCGGCCTCAC CCTGAGAGAG TACCATTCTT 1100
   GGTTGCCGAA ACACCAAGGG CCCGACACAC CCTAGATGAA TTAAACCCAC 1150
   AGAAATCCAA ACACCAAGGC GTACGGAAGG CAAAGTGGCA TTTGGGGATT 1200
   CGAAGTCAAA GCCGACCCAA TGACATCATG GCAGAAGTGT GTAGAGCAAT 1250
   CAAGCAGTTG GACTATGAAT GGAAGGTTGT AAACCCCTAT TATTTGCGTG 1300
   TGCGAAGGAA GAACCCTGTG ACAAGCACAT TTTCCAAAAT GAGTCTACAG 1350
   CTATACCAAG TGGATAGTAG GACTTACTTA TTGGATTTCC GAAGTATTGA 1400
   TGATGAGATT ACAGAAGCCA AATCAGGGAC TGCTACTCCA CAGAGATCGG 1450
   GATCCATCAG CAACTATCGA TCTTGCCAAA GGAGCGACTC CGACGCCGAG 1500
   GCTCAAGGAA AGCCCTCAGA AGTCTCTCTT ACCTCATCCG TGACCTCCCT 1550
   CGACTCCTCT CCTGTTGACG TAGCTCCAAG ACCAGGAAGT CACACGATAG 1600
   AATTTTTTGA AATGTGTGCA AATCTAATTA AAATTCTTGC ACAGTAA    1647
(2) INFORMATION FOR SEQ ID NO: 45:
   (i) SEQUENCE CHARACTERISTICS:
      (A) LENGTH: 20
      (B) TYPE: Amino Acid
      (D) TOPOLOGY: Linear
   (xi) SEQUENCE DESCRIPTION: SEQ ID NO: 45:
   Asp Phe Tyr Leu Ala Thr Ser Pro Pro Asp Ser Phe Leu Asp Asp
   1               5                   10                  15
   His His Leu Thr Arg
                   20
(2) INFORMATION FOR SEQ ID NO: 46:
   (i) SEQUENCE CHARACTERISTICS:
      (A) LENGTH: 12
      (B) TYPE: Amino Acid
      (D) TOPOLOGY: Linear
   (xi) SEQUENCE DESCRIPTION: SEQ ID NO: 46:
   Pro Leu Ser Arg Thr Leu Ser Val Ala Ala Lys Lys
   1               5                   10
(2) INFORMATION FOR SEQ ID NO: 47:
   (i) SEQUENCE CHARACTERISTICS:
      (A) LENGTH: 13
      (B) TYPE: Amino Acid
      (D) TOPOLOGY: Linear
   (xi) SEQUENCE DESCRIPTION: SEQ ID NO: 47:
   Leu Lys Lys Leu Thr Leu Arg Ala Ser Phe Ser Ala Gln
   1               5                   10
(2) INFORMATION FOR SEQ ID NO: 48:
   (i) SEQUENCE CHARACTERISTICS:
      (A) LENGTH: 14
      (B) TYPE: Amino Acid
      (D) TOPOLOGY: Linear
   (xi) SEQUENCE DESCRIPTION: SEQ ID NO: 48:
   Pro Gly Leu Lys Pro His Pro Glu Arg Met Pro Pro Leu Ile
   1               5                   10
(2) INFORMATION FOR SEQ ID NO: 49:
   (i) SEQUENCE CHARACTERISTICS:
      (A) LENGTH: 15
      (B) TYPE: Amino Acid
      (D) TOPOLOGY: Linear
   (xi) SEQUENCE DESCRIPTION: SEQ ID NO: 49:
   His Met Arg Ser Ala Met Ser Gly Leu His Leu Val Lys Arg Arg
   1               5                   10                  15
(2) INFORMATION FOR SEQ ID NO: 50:
   " (i) SEQUENCE CHARACTERISTICS:
      (A) LENGTH: 20
      (B) TYPE: Amino Acid
      (D) TOPOLOGY: Linear
   (xi) SEQUENCE DESCRIPTION: SEQ ID NO: 50:
   Asp Phe Tyr Leu Ala Thr Ser Pro Pro Asp Ser Phe Leu Asp Asp
   1               5                   10                  15
   His His Leu Thr Arg
                   20
(2) INFORMATION FOR SEQ ID NO: 51:
   (i) SEQUENCE CHARACTERISTICS:
      (A) LENGTH: 15
      (B) TYPE: Amino Acid
      (D) TOPOLOGY: Linear
   (xi) SEQUENCE DESCRIPTION: SEQ ID NO: 51:
   Met Asp Asp Ser Ala Met His Ile Pro Pro Gly Leu Lys Pro His
   1               5                   10                  15
(2) INFORMATION FOR SEQ ID NO: 52:
   (i) SEQUENCE CHARACTERISTICS:
      (A) LENGTH: 17
      (B) TYPE: Amino Acid
      (D) TOPOLOGY: Linear
   (xi) SEQUENCE DESCRIPTION: SEQ ID NO: 52:
   Val Val Asp Ile Tyr Ser Lys Phe Asp Val Ile Asn Leu Ala Ala
   1               5                   10                  15
   Glu Lys
(2) INFORMATION FOR SEQ ID NO: 53:
   (i) SEQUENCE CHARACTERISTICS:
      (A) LENGTH: 19
      (B) TYPE: Amino Acid
      (D) TOPOLOGY: Linear
   (xi) SEQUENCE DESCRIPTION: SEQ ID NO: 53:
   Gly Cys Gly Gly Ala Thr Cys Cys Gly Thr Asn Gly Ala Tyr Ala
   1               5                   10                  15
   Thr His Thr Ala
(2) INFORMATION FOR SEQ ID NO: 54:
   (i) SEQUENCE CHARACTERISTICS:
      (A) LENGTH: 22
      (B) TYPE: Amino Acid
      (D) TOPOLOGY: Linear
   (xi) SEQUENCE DESCRIPTION: SEQ ID NO: 54:
   Cys Gly Gly Ala Ala Thr Thr Cys Tyr Thr Thr Tyr Thr Cys Asn
   1               5                   10                  15
   Gly Cys Asn Gly Cys Asn Ala
                   20
(2) INFORMATION FOR SEQ ID NO: 55:
   (i) SEQUENCE CHARACTERISTICS:
      (A) LENGTH: 8
      (B) TYPE: Amino Acid
      (D) TOPOLOGY: Linear
   (xi) SEQUENCE DESCRIPTION: SEQ ID NO: 55:
   Lys Phe Asp Val Ile Asn Leu Ala
   1               5
(2) INFORMATION FOR SEQ ID NO: 56:
   (i) SEQUENCE CHARACTERISTICS:
      (A) LENGTH: 23
      (B) TYPE: Amino Acid
      (D) TOPOLOGY: Linear
   (xi) SEQUENCE DESCRIPTION: SEQ ID NO: 56:
   Ala Ala Arg Thr Thr Tyr Gly Ala Tyr Gly Thr Asn Ala Thr His
   1               5                   10                  15
   Ala Ala Tyr Cys Thr Asn Gly Cys
                   20
(2) INFORMATION FOR SEQ ID NO: 57:
   (i) SEQUENCE CHARACTERISTICS:
      (A) LENGTH: 23
      (B) TYPE: Amino Acid
      (D) TOPOLOGY: Linear
   (xi) SEQUENCE DESCRIPTION: SEQ ID NO: 57:
   Ala Ala Arg Thr Thr Tyr Gly Ala Tyr Gly Thr Asn Ala Thr His
   1               5                   10                  15
   Ala Ala Tyr Thr Thr Arg Gly Cys
                   20
(2) INFORMATION FOR SEQ ID NO: 58:
   (i) SEQUENCE CHARACTERISTICS:
      (A) LENGTH: 23
      (B) TYPE: Amino Acid
      (D) TOPOLOGY: Linear
   (xi) SEQUENCE DESCRIPTION: SEQ ID NO: 58:
   Cys Thr Cys Cys Ala Ala Gly Thr Thr Thr Gly Ala Thr Gly Thr
   1               5                   10                  15
   Thr Ala Thr Cys Ala Ala Cys Cys
                   20
(2) INFORMATION FOR SEQ ID NO: 59:
   (i) SEQUENCE CHARACTERISTICS:
      (A) LENGTH: 6
      (B) TYPE: Amino Acid
      (D) TOPOLOGY: Linear
   (xi) SEQUENCE DESCRIPTION: SEQ ID NO: 59:
   Glu Glu Leu Gln Ile Gly
   1               5
(2) INFORMATION FOR SEQ ID NO: 60:
   (i) SEQUENCE CHARACTERISTICS:
      (A) LENGTH: 7
      (B) TYPE: Amino Acid
      (D) TOPOLOGY: Linear
   (xi) SEQUENCE DESCRIPTION: SEQ ID NO: 60:
   Phe Pro Lys Pro Glu Phe Met
   1               5
(2) INFORMATION FOR SEQ ID NO: 61:
   (i) SEQUENCE CHARACTERISTICS:
      (A) LENGTH: 1978
      (B) TYPE: Nucleic acid
      (C) STRANDEDNESS: Single
      (D) TOPOLOGY: Linear
   (iv) ANTI-SENSE: No
   (xi) SEQUENCE DESCRIPTION: SEQ ID NO: 61:
   CTCGCTGCGG TCCAAGCAGG TAAAGCGGGG CTCGGCGAAC GCGCGCGACC 50
   CGAGGGGCGT GGTCCGCGGT CCCGGGGGTC CCGGCCCGGC CCTTCCCGCT 100
   TCCCTGTGTC CCCGCAGACA CTTCGCCATG GGCAATACGA GCAGCGAGCG 150
   CGCCGCGCTG GAGCGGCAGG CTGGCCATAA GACGCCGCGG AGGGACAGCT 200
   CGGAGGGCAC CAAGGATGGG GACAGGCCCA AGATCCTGAT GGACAGCCCC 250
   GAAGACGCCG ACATCTTCCA CACCGAGGAA ATGAAGGCTC CAGAGAAGGA 300
   GGAGTTCCTG GCGTGGCAGC ACGACCTCGA GGTGAATGAG AAAGCCCCCG 350
   CCCAGGCTCG GCCCACCGTA TTTCGATGGA CAGGGGGTGG AAAGGAGGTC 400
   TACTTGTCTG GATCCTTCAA CAACTGGAGC AAATTGCCCC TCACTAGAAG 450
   CCAAAACAAC TTCGTAGCCA TCCTGGACCT NCCGGAAGGA GAGCATCAGT 500
   ACAAGTTCTT TGTGGATGGC CAGTGGACCC ACGATCCTTC CGAGCCAATA 550
   GTAACCAGCC AGCTTGGCAC AGTTAACAAC ATCATTCAAG TGAAGAAAAC 600
   TGACTTTGAA GTATTTGATG CTTTAATGGT GGATTCCCAA AAGTGCTCCG 650
   ATGTATCTGA GCTGTCCAGT TCCCCCCCAG GACCCTACCA CCAGGAGCCT 700
   TACATCTCTA AACCAGAGGA GCGGTTCAAG GCCCCGCCCA TCCTCCCGCC 750
   TCACCTGCTG CAGGTCATCT TGAACAAGGA CACGGGCATC TCTTGTGATC 800
   CAGCGCTGCT TCCGGAGCCC AACCACGTCA TGCTGAACCA CCTCTATGCA 850
   CTCTCTATCA AGGATGGAGT GATGGTGCTC AGTGCGACCC ATCGGTACAA 900
   GAAAAAGTAC GTCACCACCC TCCTCTACAA GCCCATATGA GAGGATGAGC 950
   CAGCCGTGGG CCACGGGACA GCAGGCGGGA GCCGCTGGGC TCTCCGTGTG 1000
   CATGCGCATC CTCACTCCGG GACATCTCAC CCCCACATAG TCCTCCTTGA 1050
   AGGTCTGTCC AGGCACAGCC AGAAATCGGA TGGACGGCAG ACCGTGGTCC 1100
   CAGCACCGCA GGCAGTGCGC CAGGCTCTAG TGCTCTAAGC ATCATCCCTC 1150
   TGCTGGCCCG AGATGTCTAC AGCCAGACCT GAATGCTGGT TCCTGCTAGA 1200
   AAACCTAGGA CAGGAACTGA AGTCACCAAA GCCCTCATCA TCCCTGCTGA 1250
   AGCCTGGCTT GGAAGAAAGC AGTGCTCGGT CTTGCCTGTC CTTCCGAATC 1300
   ACAGCAGTAG ATTGTAGACT CCATGGAATT TCAGTGTCCA ATTTCCAGAT 1350
   GCAGCTTCGC AATCGATTCC TGACACTGTG CACTGAGACC TTCTTAACCA 1400
   GAGTGGCTGG CTGTCCACTC TCACTTAAGG CAATAAGTCA CCAGGACGAG 1450
   ACTATAGGTC ATGTGACTAC TGAGCAATAA TCGTTCTCAN ACAGACATCA 1500
   GAAACCACTG CCATTTCTCC ATCAAGCCAG ACGATCCTGA GGACTGACCA 1550
   CCATGGGAGG TTGTCCACCT TATTTCAGTT GCAGTGTTGG CCATGTTACC 1600
   GTGACAACCT GGTCGAAGTG CCCGCCCTCT TTTTAGTTCT AGCACGTGCT 1650
   ACTCAGCTGG GGGCCGTGTC TCCAGTGAGC AGAGAGTGTA CACGGTGGTT 1700
   ACTATTGCCT GATCCTAAGA GAGCTTGGCA CCCTGCGGCA GACTGCTAGG 1750
   TTCCAGCAGG GTTGGCACGA GTGAACCTAT GTGTGCTCAG TGTGATTTCC 1800
   ACAGTGATGT CACAGACGTG CCCATTGGTA CAGGCTCCTG TCACCTGTCA 1850
   GCATAGGTAG GCACAAGCTC TGTGGTGTCC GCTATTTGGT TAAACCTGAG 1900
   TTTTGGGTAC CTTTTGTTAC TGTTTTCAAA ACACGGACTT GCTGTCATCT 1950
   TGATGTACAA GTTTCAATAA AGCTTTGG                         1978
(2) INFORMATION FOR SEQ ID NO: 62:
   (i) SEQUENCE CHARACTERISTICS:
      (A) LENGTH: 270
      (B) TYPE: Amino Acid
      (D) TOPOLOGY: Linear
   (xi) SEQUENCE DESCRIPTION: SEQ ID NO: 62:
   Met Gly Asn Thr Ser Ser Glu Arg Ala Ala Leu Glu Arg Gln Ala
   1               5                   10                  15
   Gly His Lys Thr Pro Arg Arg Asp Ser Ser Glu Gly Thr Lys Asp
                   20                  25                  30
   Gly Asp Arg Pro Lys Ile Leu Met Asp Ser Pro Glu Asp Ala Asp
                   35                  40                  45
   Ile Phe His Thr Glu Glu Met Lys Ala Pro Glu Lys Glu Glu Phe
                   50                  55                  60
   Leu Ala Trp Gln His Asp Leu Glu Val Asn Glu Lys Ala Pro Ala
                   65                  70                  75
   Gln Ala Arg Pro Thr Val Phe Arg Trp Thr Gly Gly Gly Lys Glu
                   80                  85                  90
   Val Tyr Leu Ser Gly Ser Phe Asn Asn Trp Ser Lys Leu Pro Leu
                   95                  100                 105
   Thr Arg Ser Gln Asn Asn Phe Val Ala Ile Leu Asp Leu Pro Glu
                   110                 115                 120
   Gly Glu His Gln Tyr Lys Phe Phe Val Asp Gly Gln Trp Thr His
                   125                 130                 135
   Asp Pro Ser Glu Pro Ile Val Thr Ser Gln Leu Gly Thr Val Asn
                   140                 145                 150
   Asn Ile Ile Gln Val Lys Lys Thr Asp Phe Glu Val Phe Asp Ala
                   155                 160                 165
   Leu Met Val Asp Ser Gln Lys Cys Ser Asp Val Ser Glu Leu Ser
                   170                 175                 180
   Ser Ser Pro Pro Gly Pro Tyr His Gln Glu Pro Tyr Ile Ser Lys
                   185                 190                 195
   Pro Glu Glu Arg Phe Lys Ala Pro Pro Ile Leu Pro Pro His Leu
                   200                 205                 210
   Leu Gln Val Ile Leu Asn Lys Asp Thr Gly Ile Ser Cys Asp Pro
                   215                 220                 225
   Ala Leu Leu Pro Glu Pro Asn His Val Met Leu Asn His Leu Tyr
                   230                 235                 240
   Ala Leu Ser Ile Lys Asp Gly Val Met Val Leu Ser Ala Thr His
                   245                 250                 255
   Arg Tyr Lys Lys Lys Tyr Val Thr Thr Leu Leu Tyr Lys Pro Ile
                   260                 265                 270
(2) INFORMATION FOR SEQ ID NO: 63:
   (i) SEQUENCE CHARACTERISTICS:
      (A) LENGTH: 1576
      (B) TYPE: Nucleic acid
      (C) STRANDEDNESS: Single
      (D) TOPOLOGY: Linear
   (iv) ANTI-SENSE: No
   (xi) SEQUENCE DESCRIPTION: SEQ ID NO: 63:
   GCGCCCTTAA AGATGGTGAG GGGGCTATGC TCTGAGTAGA AGGTGGTGAC 50
   CTCCAGGAGC GGTGGGATGA TGAGGGCCCG GGCGCCTCTT GCAATGGAGA 100
   CGGTCATTTC TTCAGATAGC TCCCCAGCTG TGGAAAATGA GCATCCTCAA 150
   GAGACCCCAG AATCCAACAA TAGCGTGTAT ACTTCCTTCA TGAAGTCTCA 200
   TCGCTGCTAT GACCTGATTC CCACAAGCTC CAAATTGGTT GTATTTGATA 250
   CGTCCCTGCA GGTGAAGAAA GCTTTTTTTG CTTTGGTGAC TAACGGTGTA 300
   CGAGCTGCCC CTTTATGGGA TAGTAAGAAG CAAAGTTTTG TGGGCATGCT 350
   GACCATCACT GATTTCATCA ATATCCTGCA CCGCTACTAT AAATCAGCGT 400
   TGGTACAGAT CTATGAGCTA GAAGAACACA AGATAGAAAC TTGGAGAGAG 450
   GTGTATCTCC AGGACTCCTT TAAACCGCTT GTCTGCATTT CTCCTAATGC 500
   CAGCTTGTTT GATGCTGTCT CTTCATTAAT TCGCAACAAG ATCCACAGGC 550
   TGCCAGTTAT TGACCCAGAA TCAGGCAATA CTTTGTACAT CCTCACCCAC 600
   AAGCGCATTC TGAAGTTCCT CAAATTGTTT ATCACTGAGT TCCCCAAGCC 650
   AGAGTTCATG TCCAAGTCTC TGGAAGAGCT ACAGATTGGC ACCTATGCCA 700
   ATATTGCTAT GGTTCGCACT ACCACCCCCG TCTATGTGGC TCTGGGGATT 750
   TTTGTACAGC ATCGAGTCTC AGCCCTGCCA GTGGTGGATG AGAAGGGGCG 800
   TGTGGTGGAC ATCTACTCCA AGTTTGATGT TATCAATCTG GCAGCAGAAA 850
   AGACCTACAA CAACCTAGAT GTATATGTGA CTAAAGCCTT GCAACATCGA 900
   TCACATTACT TTGAGGGTGT TCTCAAGTGC TACCTGCATG AGACTCTGGA 950
   GACCATCATC AACAGGCTAG TGGAAGCAGA GGTTCACCGA CTTGTAGTGG 1000
   TGGATGAAAA TGATGTGGTC AAGGGAATTG TATCACTGTC TGACATCCTG 1050
   CAGGCCCTGG TGCTCACAGG TGGAGAGAAG AAGCCCTGAG CTGGGGAAGG 1100
   GGTCATGCAG CACCAGGGGA TATGCCCAAC TCACTGCCTG CTGGAAGCTC 1150
   TGTGGGAATC AGATGAAACT TGAGGGAATT GTGACTCTGT TCCCTGTTCA 1200
   GGGTCCCCTG CCCTTCTATC TGGGAGCTAG GGAAGGTATG GGGGAGGAAA 1250
   GAGAATGGAT TTATAGCTAC CCTTACCCTC ACACATACAC TTGAAAAAAC 1300
   TTTCAGCCTA GCCAGTTCTA GCCCCTGTCC TCTTAGATAT ATCCCCCTTT 1350
   CTGGGTGAAC TATAGGCTCT GTGCCTCTCA GACAAATTCT GATCTCTAAG 1400
   AGATCCCCAG ACCTCACTTG CCTCTGCCTC CATCTTGGCC CTGATTCAAC 1450
   CCTAAGATAA TAGCACAACA AAATTCTTCA TAAAGATATT TTTATTCACC 1500
   TGTTCCGTGC TATATGGAGG AGGCCAAGTC CATTTAGTGA CATTTCTTCC 1550
   CATAATGTGA GTGGGGAGGA TTGTGG                           1576
(2) INFORMATION FOR SEQ ID NO: 64:
   (i) SEQUENCE CHARACTERISTICS:
      (A) LENGTH: 331
      (B) TYPE: Amino Acid
      (D) TOPOLOGY: Linear
   (xi) SEQUENCE DESCRIPTION: SEQ ID NO: 64:
   Met Glu Thr Val Ile Ser Ser Asp Ser Ser Pro Ala Val Glu Asn
   1               5                   10                  15
   Glu His Pro Gln Glu Thr Pro Glu Ser Asn Asn Ser Val Tyr Thr
                   20                  25                  30
   Ser Phe Met Lys Ser His Arg Cys Tyr Asp Leu Ile Pro Thr Ser
                   35                  40                  45
   Ser Lys Leu Val Val Phe Asp Thr Ser Leu Gln Val Lys Lys Ala
                   50                  55                  60
   Phe Phe Ala Leu Val Thr Asn Gly Val Arg Ala Ala Pro Leu Trp
                   65                  70                  75
   Asp Ser Lys Lys Gln Ser Phe Val Gly Met Leu Thr Ile Thr Asp
                   80                  85                  90
   Phe Ile Asn Ile Leu His Arg Tyr Tyr Lys Ser Ala Leu Val Gln
                   95                  100                 105
   Ile Tyr Glu Leu Glu Glu His Lys Ile Glu Thr Trp Arg Glu Val
                   110                 115                 120
   Tyr Leu Gln Asp Ser Phe Lys Pro Leu Val Cys Ile Ser Pro Asn
                   125                 130                 135
   Ala Ser Leu Phe Asp Ala Val Ser Ser Leu Ile Arg Asn Lys Ile
                   140                 145                 150
   His Arg Leu Pro Val Ile Asp Pro Glu Ser Gly Asn Thr Leu Tyr
                   155                 160                 165
   Ile Leu Thr His Lys Arg Ile Leu Lys Phe Leu Lys Leu Phe Ile
                   170                 175                 180
   Thr Glu Phe Pro Lys Pro Glu Phe Met Ser Lys Ser Leu Glu Glu
                   185                 190                 195
   Leu Gln Ile Gly Thr Tyr Ala Asn Ile Ala Met Val Arg Thr Thr
                   200                 205                 210
   Thr Pro Val Tyr Val Ala Leu Gly Ile Phe Val Gln His Arg Val
                   215                 220                 225
   Ser Ala Leu Pro Val Val Asp Glu Lys Gly Arg Val Val Asp Ile
                   230                 235                 240
   Tyr Ser Lys Phe Asp Val Ile Asn Leu Ala Ala Glu Lys Thr Tyr
                   245                 250                 255
   Asn Asn Leu Asp Val Ser Val Thr Lys Ala Leu Gln His Arg Ser
                   260                 265                 270
   His Tyr Phe Glu Gly Val Leu Lys Cys Tyr Leu His Glu Thr Leu
                   275                 280                 285
   Glu Thr Ile Ile Asn Arg Leu Val Glu Ala Glu Val His Arg Leu
                   290                 295                 300
   Val Val Val Asp Glu Asn Asp Val Val Lys Gly Ile Val Ser Leu
                   305                 310                 315
   Ser Asp Ile Leu Gln Ala Leu Val Leu Thr Gly Gly Glu Lys Lys
                   320                 325                 330
   Pro
(2) INFORMATION FOR SEQ ID NO: 65:
   (i) SEQUENCE CHARACTERISTICS:
      (A) LENGTH: 2761
      (B) TYPE: Nucleic acid
      (C) STRANDEDNESS: Single
      (D) TOPOLOGY: Linear
   (iv) ANTI-SENSE: No
   (xi) SEQUENCE DESCRIPTION: SEQ ID NO: 65:
   CCCGGGGAGG CCGCGCGCCG AACATGGCTG AGAAGCAGAA GCACGACGGG CGTGTGAAGA     60
   TCGGACACTA CGTGCTGGGG GACACCCTGG GCGTCGGCAC CTTCGGCAAA GTGAAGATTG    120
   GAGAACATCA ATTGACAGGC CATAAAGTGG CAGTTAAGAT CTTAAATAGA CAGAAGATTC    180
   GCAGTTTAGA TGTTGTTGGA AAAATAAAAC GAGAAATTCA AAATCTTAAA CTCTTTCGTC    240
   ATCCTCATAT TATCAAACTC TACCAAGTGA TCAGCACTCC AACAGACTTT TTTATGGTAA    300
   TGGAATATGT GTCTGGAGGT GAATTGTTCG ACTACATCTG TAAACACGGG AGGGTTGAAG    360
   AGGTGGAAGC TCGCCGGCTC TTCCAGCAGA TTCTGTCTGC CGTGGACTAC TGTCACAGGC    420
   ACATGGTTGT CCACAGGGAC CTGAAGCCAG AGAACGTGTT GCTGGACGCC CAGATGAATG    480
   CTAAGATAGC TGACTTCGGA CTCTCTAATA TGATGTCAGA TGGTGAATTT CTACGAACTA    540
   GCTGTGGATC GCCAAATTAT GCAGCACCGG AGGTCATCTC AGGAAGGCTG TATGCGGGTC    600
   CTGAGGTTGA TATCTGGAGC TGTGGTGTTA TCCTGTATGC CCTTCTCTGT GGCACCCTCC    660
   CGTTCGACGA TGAGCACGTG CCTACGCTCT TTAAGAAGAT CCGAGGGGGT GTGTTCTACA    720
   TCCCGGAGTA TCTCAACCGT TCTATTGCCA CTCTGCTGAT GCACATGCTG CAGGTGGACC    780
   CCTTGAAGCG AGCAACTATC AAAGACATAC GAGAGCATGA ATGGTTTAAA CAGGATTTGC    840
   CCAGTTACCT CTTTCCTGAA GACCCCTCCT ATGATGCTAA CGTCATTGAT GATGAGGCTG    900
   TGAAAGAAGT ATGTGAAAAA TTTGAGTGTA CAGAATCAGA AGTGATGAAC AGTTTATACA    960
   GTGGTGACCC TCAAGACCAG CTCGCAGTGG CTTATCATCT CATCATTGAC AATCGGAGAA   1020
   TAATGAACCA AGCCAGTGAG TTCTACCTCG CCTCCAGTCC TCCAACGGGT TCCTTCATGG   1080
   ACGATATGGC CATGCACATT CCCCCCGGCC TGAAACCACA TCCTGAAAGG ATGCCACCTC   1140
   TCATAGCAGA CAGCCCCAAA GCACGCTGTC CACTGGATGC ACTCAACACA ACTAAGCCCA   1200
   AATCTTTAGC TGTGAAAAAA GCCAAGTGGC ACCTTGGGAT CCGAAGCCAG AGCAAACCAT   1260
   ACGACATTAT GGCGGAGGTG TACCGAGCTA TGAAGCAGCT GGACTTTGAA TGGAAGGTAG   1320
   TGAATGCATA CCATCTTCGA GTAAGAAGAA AAAACCCAGT GACTGGCAAT TACGTGAAAA   1380
   TGAGCTTACA GCTTTACCTG GTTGACAATC GGAGCTATCT TCTAGACTTT AAAAGCATCG   1440
   ATGATGAGGT GGTGGAGCAG AGGTCTGGTT CTTCAACACC TCAGCGCTCC TGTTCTGCTG   1500
   CCGGCCTCCA CAGACCTCGG TCAAGTGTCG ATTCCAGCAC AGCCGAGAAC CATTCACTGT   1560
   CTGGCTCTCT CACTGGTTCT TTGACTGGCA GAACTTTGTC CTCCGCTTCC CCGCGCCTGG   1620
   GCAGTCATAC CATGGATTTT TTTGAAATGT GCGCCAGTCT TATCACTGCT TTAGCCCGTT   1680
   GATAACCCAC CACCGGTCTC TGTCTTTCTG TTACCGCACT GTGAAATCAC ATACACTCTT   1740
   CAAATTATTA CCGCACTCTC GGGTACCACA GGCTCTGCAA TAGAAGTTAT GTGAACATTC   1800
   CCAGGTGACA TGCAGTGCTG CTGGAAACAC AGAAATCTGG CCTTCTGTTT ACTTTTAGAA   1860
   CTCTGTAACT CTGCTGTGCC TATGATAGGT ATCAATAGCT AGGAACGGCT GAGTGCTGGT   1920
   GAAGCTTGTT AACTTACACC CGTGAATTCA CTACACATGG TGAGCACACC TCACTGATGA   1980
   ACCCGCTGAT CTCGGGGTGG TTCGGTGGGA CCGCCTTCCT TCACGTTTAG TTCATGTAAA   2040
   TCCTGTTTGC CTCCTAAATT TCCATAGGTG TCAGGCTGTC TAGGCACTCT TGGACAAGAA   2100
   GATTCAGAAA TAGAGTAACT GTCAGTGAAA TATTATTTAA ATGTAGAAAT CCGAAATCCT   2160
   GTCCCCTTAA ATATCAGAAA CCAAAAGTCT TTTTTAATAC TTTCTGCAAA TACTGCCTAG   2220
   TATTAGCCAT ACAGACTGTG TTTCTGATAA TAGGAGCCCA GTCTCCTAGC TTCCTGACAT   2280
   TTGTGCAAGG CCCCTAGTTA AATCCCACTA CCACAAAGCA ATATAAAACC ATGCAAAGTT   2340
   AGTGCATAGT TAAAGGAAAC AGGCAGATAC CAACAGTCTC TTAAAAGGAA ATCTATTCTT   2400
   TGATCTCATT TGTGTTTATG AGACTGGGTA GCTGGGGGCT TGGGGGCGGG CTGGGAGTCA   2460
   CAGCCCTTGG GCATCTTTGC TAATCCACTT GAACTTTGTT ATTGATGCCG AGTCCTCTCT   2520
   CTTCCTCAGT AGCCACTGTC TTTGCTCATT GCTTTTCCCT TTTTTAACTC CTTTAGATCA   2580
   AAACCTTGCT TTGGCCATAG GGTCTTTAAA TACTTTCAAA GCTTGATCTG CTGTGACCTT   2640
   CACTGTTGAA CCTGATTGGA CAGGGAAGCC TTAAATATTT AAAAGTATAT TCTCCTGGAA   2700
   TAACTACATG TGTTGTTTAC ATATATATAC ATATGTATTG ACACATCAGT GCTTTTAATC   2760
   G                                                                   2761
(2) INFORMATION FOR SEQ ID NO: 66:
   (i) SEQUENCE CHARACTERISTICS:
      (A) LENGTH: 1783
      (B) TYPE: Nucleic acid
      (C) STRANDEDNESS: Single
      (D) TOPOLOGY: Linear
   (iv) ANTI-SENSE: No
   (xi) SEQUENCE DESCRIPTION: SEQ ID NO: 66:
   GATGGCTGAG AAGCAGAAGC ACGACGGGCG GGTGAAGATC GGACACTACG TGCTGGGCGA     60
   CACGCTGGGC GTCGGCACCT TCGGCAAAGT GAAGATTGGA GAACATCAAT TAACAGGCCA    120
   TAAAGTGGCA GTTAAAATCT TAAATAGACA GAAGATTCGC AGTTTAGATG TTGTTGGAAA    180
   AATAAAACGA GAAATTCAAA ATCTAAAACT CTTTCGTCAT CCTCATATTA TCAAACTATA    240
   CCAGGTGATC AGCACTCCAA CAGATTTTTT TATGGTAATG GAATATGTGT CTGGAGGTGA    300
   ATTATTTGAC TACATCTGTA AGCATGGACG GGTTGAAGAG ATGGAAGCCA GGCGGCTCTT    360
   TCAGCAGATT CTGTCTGCTG TGGATTACTG TCATAGGCAT ATGGTTGTTC ATCGAGACCT    420
   GAAACCAGAG AATGTCCTGT TGGATGCACA CATGAATGCC AAGATAGCCG ATTTCGGATT    480
   ATCTAATATG ATGTCAGATG GTGAATTTCT GAGAACTAGT TGCGGATCTC CAAATTATGC    540
   AGCACCTGAA GTCATCTCAG GCAGATTGTA TGCAGGTCCT GAAGTTGATA TCTGGAGCTG    600
   TGGTGTTATC TTGTATGCTC TTCTTTGTGG CACCCTCCCA TTTGATGATG AGCATGTACC    660
   TACGTTATTT AAGAAGATCC GAGGGGGTGT CTTTTATATC CCAGAATATC TCAATCGTTC    720
   TGTCGCCACT CTCCTGATGC ATATGCTGCA GGTTGACCCA CTGAAACGAG CAACTATCAA    780
   AGACATAAGA GAGCATGAAT GGTTTAAACA AGATTTGCCC AGTTACTTAT TTCCTGAAGA    840
   CCCTTCCTAT GATGCTAACG TCATTGATGA TGAGGCTGTG AAAGAAGTGT GTGAAAAATT    900
   TGAATGTACA GAATCAGAAG TAATGAACAG TTTATATAGT GGTGACCCTC AAGACCAGCT    960
   TGCAGTGGCT TATCATCTTA TCATTGACAA TCGGAGAATA ATGAACCAAG CCAGTGAGTT   1020
   CTATCTCGCC TCTAGTCCTC CATCTGGTTC TTTTATGGAT GATAGTGCCA TGCATATTCC   1080
   CCCAGGCCTG AAACCTCATC CAGAAAGGAT GCCACCTCTT ATAGCAGACA GCCCCAAAGC   1140
   AAGATGTCCA TTGGATGCAC TGAATACGAC TAAGCCCAAA TCTTTAGCTG TGAAAAAAGC   1200
   CAAGTGGCAT CTTGGAATCC GAAGTCAGAG CAAACCGTAT GACATTATGG CTGAAGTTTA   1260
   CCGAGCTATG AAGCAGCTGG ATTTTGAATG GAAGGTAGTG AATGCATACC ATCTTCGTGT   1320
   AAGAAGAAAA AATCCAGTGA CTGGCAATTA CGTGAAAATG AGCTTACAAC TTTACCTGGT   1380
   TGATAACAGG AGCTATCTTT TGGACTTTAA AAGCATTGAT GATGAAGTAG TGGAGCAGAG   1440
   ATCTGGTTCC TCAACACCTC AGCGTTCCTG TTCTGCTGCT GGCTTACACA GACCAAGATC   1500
   AAGTTTTGAT TCCACAACTG CAGAGAGCCA TTCACTTTCT GGCTCTCTCA CTGGCTCTTT   1560
   GACCGGAAGC ACATTGTCTT CAGTTTCACC TCGCCTGGGC AGTCACACCA TGGATTTTTT   1620
   TGAAATGTGT GCCAGTCTGA TTACTACTTT AGCCCGTTGA TCTGTCTCTA GTTTCTTTCT   1680
   GTTATTGCAC TATGAAAATC AGTTATATTC TTTAAATTTT TATCTTACTT TTGGATAATA   1740
   TCCATCCAGC TTGGACTTAA CCAGGCTGAA CTTGCTCAAA AGG                     1783
(2) INFORMATION FOR SEQ ID NO: 67:
   (i) SEQUENCE CHARACTERISTICS:
      (A) LENGTH: 45
      (B) TYPE: Nucleic acid
      (C) STRANDEDNESS: Single
      (D) TOPOLOGY: Linear
   (iv) ANTI-SENSE: No
   (xi) SEQUENCE DESCRIPTION: SEQ ID NO: 67:
   GATGATCACC ATTTAACTCG GCCTCACCCT GAGAGAGTAC CATTC     45